# EUROPEAN PATENT SPECIFICATION

(11) **EP 3 570 875 B1**
(45) Date of publication and mention of the grant of the patent: **06.07.2022**
(21) Application number: 18705843.3
(22) Date of filing: 05.02.2018
(51) Int. Cl.: C07K 14/62, C07K 14/705, A61K 38/00

(54) **PEPTIDE MODULATORS OF THE INTERACTION BETWEEN HUMAN C-PEPTIDE AND HUMAN ELASTIN RECEPTOR FOR THERAPEUTIC USE**
PEPTIDMODULATOREN DER WECHSELWIRKUNG ZWISCHEN MENSCHLICHEM C-PEPTID UND MENSCHLICHEM ELASTINREZEPTOR ZUR THERAPEUTISCHEN VERWENDUNG
MODULATEURS PEPTIDIQUES DE L'INTERACTION ENTRE LE C-PEPTIDE HUMAIN ET LE RÉCEPTEUR D'ELASTINE HUMAIN À USAGE THERAPEUTIQUE

(30) Priority: 06.02.2017 EP 17154889
(43) Date of publication of application: 27.11.2019
(73) Proprietor: Biotempt B.V., 2514 EN Den Haag (NL)
(72) Inventor: WENSVOORT, Gert, 7958 NZ Koekange (NL)
(74) Representative: V.O.
(86) International application number: PCT/EP2018/052822
(87) International publication number: WO 2018/141969

(56) References cited:
- WO-A1-2005/118638
- US-A1- 2007 082 842
- QA'ATY NOUR ET AL: "Synthetic ligands of the elastin receptor induce elastogenesis in human dermal fibroblasts via activation of their IGF-1 receptors", JOURNAL OF DERMATOLOGICAL SCIENCE, vol. 80, no. 3, 9 October 2015 (2015-10-09), pages 175-185, XP029348101, ISSN: 0923-1811, DOI: 10.1016/J.JDERMSCI.2015.10.001
- ROBINET ARNAUD ET AL: "Elastin-derived peptides enhance angiogenesis by promoting endothelial cell migration and tubulogenesis through upregulation of MT1-MMP", JOURNAL OF CELL SCIENCE, CAMBRIDGE UNIVERSITY PRESS, LONDON, GB, vol. 118, no. 2, 15 January 2005 (2005-01-15), pages 343-356, XP002414909, ISSN: 0021-9533, DOI: 10.1242/JCS.01613
- C. KAWECKI ET AL: "Elastin-Derived Peptides Are New Regulators of Thrombosis", ARTERIOSCLEROSIS, THROMBOSIS, AND VASCULAR BIOLOGY., vol. 34, no. 12, 1 December 2014 (2014-12-01), pages 2570-2578, XP055472863, US ISSN: 1079-5642, DOI: 10.1161/ATVBAHA.114.304432
- S. BLAISE ET AL: "Elastin-Derived Peptides Are New Regulators of Insulin Resistance Development in Mice", DIABETES, vol. 62, no. 11, 6 August 2013 (2013-08-06), pages 3807-3816, XP055461109, US ISSN: 0012-1797, DOI: 10.2337/db13-0508
- CHARLOTTE BLANCHEVOYE ET AL: "Interaction between the Elastin Peptide VGVAPG and Human Elastin Binding Protein", JOURNAL OF BIOLOGICAL CHEMISTRY, vol. 288, no. 2, 11 January 2013 (2013-01-11), pages 1317-1328, XP055461117, US ISSN: 0021-9258, DOI: 10.1074/jbc.M112.419929
- LEONARD E. GROSSO ET AL: "Peptide sequences selected by BA4, a tropoelastin-specific monoclonal antibody, are ligands for the 67-kilodalton bovine elastin receptor", BIOCHEMISTRY, vol. 32, no. 48, 1 December 1993 (1993-12-01), pages 13369-13374, XP055003161, ISSN: 0006-2960, DOI: 10.1021/bi00211a052
- MAURICE PASCAL ET AL: "Elastin fragmentation and atherosclerosis progression: The elastokine concept", TRENDS IN CARDIOVASCULAR MEDICINE, vol. 23, no. 6, 2 April 2013 (2013-04-02), pages 211-221, XP028672403, ISSN: 1050-1738, DOI: 10.1016/J.TCM.2012.12.004

### SEQUENCE LISTING

<110> Biotempt B.V. Wensvoort Gert
<120> PEPTIDE AGONISTS AND ANTAGONISTS OF C PEPTIDES INTERACTION WITH THE ELASTIN
   RECEPTOR
<130> 2017.004 WO 1
<150> EP17154889
   <151> 2017-02-06
<160> 216
<170> BiSSAP 1.3.6
<210> 1
   <211> 31
   <212> PRT
   <213> Homo sapiens
<400> 1
   Glu Ala Glu Asp Leu Gln Val Gly Gln Val Glu Leu Gly Gly Gly Pro
   1               5                   10                  15
   Gly Ala Gly Ser Leu Gln Pro Leu Ala Leu Glu Gly Ser Leu Gln
               20                  25                  30
<210> 2
   <211> 12
   <212> PRT
   <213> Homo sapiens
<400> 2
   Glu Ala Glu Asp Leu Gln Val Gly Gln Val Glu Leu
   1 5 10
<210> 3
   <211> 13
   <212> PRT
   <213> Homo sapiens
<400> 3
   Gly Gly Gly Pro Gly Ala Gly Ser Leu Gln Pro Leu Ala
   1 5 10
<210> 4
   <211> 6
   <212> PRT
   <213> Homo sapiens
<400> 4
   Leu Glu Gly Ser Leu Gln
   1 5
<210> 5
   <211> 4
   <212> PRT
   <213> Homo sapiens
<400> 5
   Glu Ala Glu Asp
   1
<210> 6
   <211> 5
   <212> PRT
   <213> Homo sapiens
<400> 6
   Glu Gly Ser Leu Gln
   1 5
<210> 7
   <211> 5
   <212> PRT
   <213> Rattus norvegicus
<400> 7
   Glu Val Ala Arg Gln
   1 5
<210> 8
   <211> 7
   <212> PRT
   <213> Homo sapiens
<400> 8
   Gly Gly Gly Pro Gly Ala Gly
   1 5
<210> 9
   <211> 9
   <212> PRT
   <213> Homo sapiens
<400> 9
   Glu Leu Gly Gly Gly Pro Gly Ala Gly
   1 5
<210> 10
   <211> 28
   <212> PRT
   <213> Homo sapiens
<400> 10
   Asp Leu Gln Val Gly Gln Val Glu Leu Gly Gly Gly Pro Gly Ala Gly
   1 5 10 15
   Ser Leu Gln Pro Leu Ala Leu Glu Gly Ser Leu Gln
   20 25
<210> 11
   <211> 27
   <212> PRT
   <213> Homo sapiens
<400> 11
   Leu Gln Val Gly Gln Val Glu Leu Gly Gly Gly Pro Gly Ala Gly Ser
   1 5 10 15
   Leu Gln Pro Leu Ala Leu Glu Gly Ser Leu Gln
   20 25
<210> 12
   <211> 25
   <212> PRT
   <213> Homo sapiens
<400> 12
   Val Gly Gln Val Glu Leu Gly Gly Gly Pro Gly Ala Gly Ser Leu Gln
   1 5 10 15
   Pro Leu Ala Leu Glu Gly Ser Leu Gln
   20 25
<210> 13
   <211> 24
   <212> PRT
   <213> Homo sapiens
<400> 13
   Gly Gln Val Glu Leu Gly Gly Gly Pro Gly Ala Gly Ser Leu Gln Pro
   1 5 10 15
   Leu Ala Leu Glu Gly Ser Leu Gln
   20
<210> 14
   <211> 20
   <212> PRT
   <213> Homo sapiens
<400> 14
   Leu Gly Gly Gly Pro Gly Ala Gly Ser Leu Gln Pro Leu Ala Leu Glu
   1 5 10 15
   Gly Ser Leu Gln
   20
<210> 15
   <211> 20
   <212> PRT
   <213> Homo sapiens
<400> 15
   Val Gly Gln Val Glu Leu Gly Gly Gly Pro Gly Ala Gly Ser Leu Gln
   1 5 10 15
   Pro Leu Ala Leu
   20
<210> 16
   <211> 19
   <212> PRT
   <213> Homo sapiens
<400> 16
   Glu Val Gly Gln Val Glu Leu Gly Gly Gly Pro Gly Ala Gly Ser Leu
   1 5 10 15
   Gln Pro Leu
<210> 17
   <211> 26
   <212> PRT
   <213> Homo sapiens
<400> 17
   Glu Ala Glu Asp Leu Gln Val Gly Gln Val Glu Leu Gly Gly Gly Pro
   1               5                   10                  15
   Gly Ala Gly Ser Leu Gln Pro Leu Ala Leu
               20                  25
<210> 18
   <211> 24
   <212> PRT
   <213> Homo sapiens
<400> 18
   Glu Ala Glu Asp Leu Gln Val Gly Gln Val Glu Leu Gly Gly Gly Pro
   1               5                   10                  15
   Gly Ala Gly Ser Leu Gln Pro Leu
               20
<210> 19
   <211> 22
   <212> PRT
   <213> Homo sapiens
<400> 19
   Leu Gln Val Gly Gln Val Glu Leu Gly Gly Gly Pro Gly Ala Gly Ser
   1               5                   10                  15
   Leu Gln Pro Leu Ala Leu
               20
<210> 20
   <211> 21
   <212> PRT
   <213> Homo sapiens
<400> 20
   Asp Leu Gln Val Gly Gln Val Glu Leu Gly Gly Gly Pro Gly Ala Gly
   1               5                   10                  15
   Ser Leu Gln Pro Leu
               20
<210> 21
   <211> 20
   <212> PRT
   <213> Homo sapiens
<400> 21
   Leu Gln Val Gly Gln Val Glu Leu Gly Gly Gly Pro Gly Ala Gly Ser
   1 5 10 15
   Leu Gln Pro Leu
   20
<210> 22
   <211> 13
   <212> PRT
   <213> Homo sapiens
<400> 22
   Leu Gly Gly Gly Pro Gly Ala Gly Ser Leu Gln Pro Leu
   1 5 10
<210> 23
   <211> 15
   <212> PRT
   <213> Homo sapiens
<400> 23
   Val Gly Gln Val Glu Leu Gly Gly Gly Pro Gly Ala Gly Ser Leu
   1 5 10 15
<210> 24
   <211> 10
   <212> PRT
   <213> Homo sapiens
<400> 24
   Gly Gly Gly Pro Gly Ala Gly Ser Leu Gln
   1 5 10
<210> 25
   <211> 19
   <212> PRT
   <213> Homo sapiens
<400> 25
   Phe Arg Ala Ala Pro Leu Gln Gly Met Leu Pro Gly Leu Leu Ala Pro
   1 5 10 15
   Leu Arg Thr
<210> 26
   <211> 15
   <212> PRT
   <213> Homo sapiens
<400> 26
   Ala Ala Pro Leu Gln Gly Met Leu Pro Gly Leu Leu Ala Pro Leu
   1 5 10 15
<210> 27
   <211> 12
   <212> PRT
   <213> Homo sapiens
<400> 27
   Leu Gln Gly Met Leu Pro Gly Leu Leu Ala Pro Leu
   1 5 10
<210> 28
   <211> 10
   <212> PRT
   <213> Homo sapiens
<400> 28
   Leu Gln Gly Met Leu Pro Gly Leu Leu Ala
   1 5 10
<210> 29
   <211> 7
   <212> PRT
   <213> Homo sapiens
<400> 29
   Leu Gln Gly Met Leu Pro Gly
   1 5
<210> 30
   <211> 8
   <212> PRT
   <213> Homo sapiens
<400> 30
   Gly Met Leu Pro Gly Leu Leu Ala
   1 5
<210> 31
   <211> 4
   <212> PRT
   <213> Homo sapiens
<400> 31
   Gln Asp Glu Ala
   1
<210> 32
   <211> 8
   <212> PRT
   <213> Homo sapiens
<400> 32
   Leu Gly Gly Gly Pro Gly Ala Gly
   1 5
<210> 33
   <211> 8
   <212> PRT
   <213> Bos taurus
<400> 33
   Leu Ala Gly Gly Pro Gly Ala Gly
   1 5
<210> 34
   <211> 6
   <212> PRT
   <213> Homo sapiens
<400> 34
   Leu Gly Gly Gly Pro Gly
   1 5
<210> 35
   <211> 6
   <212> PRT
   <213> Bos taurus
<400> 35
   Leu Ala Gly Gly Pro Gly
   1 5
<210> 36
   <211> 5
   <212> PRT
   <213> Homo sapiens
<400> 36
   Leu Gly Gly Gly Pro
   1 5
<210> 37
   <211> 5
   <212> PRT
   <213> Bos taurus
<400> 37
   Leu Ala Gly Gly Pro
   1 5
<210> 38
   <211> 4
   <212> PRT
   <213> Homo sapiens
<400> 38
   Gly Gly Gly Pro
   1
<210> 39
   <211> 4
   <212> PRT
   <213> Artificial Sequence
<220>
   <223> Retro sequence peptide
<400> 39
   Gly Ala Gly Pro
   1
<210> 40
   <211> 5
   <212> PRT
   <213> Homo sapiens
<400> 40
   Gly Gly Gly Pro Gly
   1 5
<210> 41
   <211> 6
   <212> PRT
   <213> Homo sapiens
<400> 41
   Val Gly Val Ala Pro Gly
   1 5
<210> 42
   <211> 10
   <212> PRT
   <213> Homo sapiens
<400> 42
   Glu Leu Gly Gly Gly Pro Gly Ala Gly Ser
   1               5                   10
<210> 43
   <211> 6
   <212> PRT
   <213> Artificial Sequence
<220>
   <223> Immunomodulatory peptide
<400> 43
   Gln Gly Gln Leu Pro Gly
   1               5
<210> 44
   <211> 6
   <212> PRT
   <213> Homo sapiens
<220>
   <223> selected from human Galectin-3
<400> 44
   Pro Gly Ala Tyr Pro Gly
   1               5
<210> 45
   <211> 6
   <212> PRT
   <213> Homo sapiens
<220>
   <223> selected from loop 2 of human beta-hCG
<400> 45
   Gln Gly Val Leu Pro Ala
   1               5
<210> 46
   <211> 5
   <212> PRT
   <213> Bos taurus
<400> 46
   Gly Ala Gly Pro Gly
   1 5
<210> 47
   <211> 7
   <212> PRT
   <213> Homo sapiens
<400> 47
   Leu Gly Gly Gly Pro Gly Ala
   1 5
<210> 48
   <211> 6
   <212> PRT
   <213> Homo sapiens
<400> 48
   Gly Gly Pro Gly Ala Gly
   1 5
<210> 49
   <211> 6
   <212> PRT
   <213> Homo sapiens
<400> 49
   Gly Gly Gly Pro Gly Ala
   1 5
<210> 50
   <211> 5
   <212> PRT
   <213> Homo sapiens
<400> 50
   Gly Gly Gly Pro Glu
   1 5
<210> 51
   <211> 5
   <212> PRT
   <213> Homo sapiens
<400> 51
   Gly Ala Ile Pro Gly
   1 5
<210> 52
   <211> 5
   <212> PRT
   <213> Homo sapiens
<400> 52
   Gly Gly Val Pro Gly
   1               5
<210> 53
   <211> 5
   <212> PRT
   <213> Homo sapiens
<400> 53
   Gly Val Ala Pro Gly
   1               5
<210> 54
   <211> 14
   <212> PRT
   <213> Homo sapiens
<400> 54
   Tyr Thr Thr Gly Lys Leu Pro Tyr Gly Tyr Gly Pro Gly Gly
   1               5                   10
<210> 55
   <211> 12
   <212> PRT
   <213> Homo sapiens
<400> 55
   Tyr Gly Ala Arg Pro Gly Val Gly Val Gly Ile Pro
   1               5                   10
<210> 56
   <211> 9
   <212> PRT
   <213> Homo sapiens
<400> 56
   Pro Gly Phe Gly Ala Val Pro Gly Ala
   1               5
<210> 57
   <211> 5
   <212> PRT
   <213> Homo sapiens
<400> 57
   Gly Val Tyr Pro Gly
   1 5
<210> 58
   <211> 5
   <212> PRT
   <213> Homo sapiens
<400> 58
   Gly Phe Gly Pro Gly
   1 5
<210> 59
   <211> 5
   <212> PRT
   <213> Homo sapiens
<400> 59
   Gly Val Leu Pro Gly
   1 5
<210> 60
   <211> 6
   <212> PRT
   <213> Homo sapiens
<400> 60
   Pro Gly Ala Ile Pro Gly
   1 5
<210> 61
   <211> 6
   <212> PRT
   <213> Homo sapiens
<400> 61
   Pro Gly Ala Val Gly Pro
   1 5
<210> 62
   <211> 6
   <212> PRT
   <213> Homo sapiens
<400> 62
   Val Gly Ala Met Pro Gly
   1 5
<210> 63
   <211> 6
   <212> PRT
   <213> Homo sapiens
<400> 63
   Val Gly Ser Leu Pro Gly
   1 5
<210> 64
   <211> 6
   <212> PRT
   <213> Homo sapiens
<400> 64
   Val Gly Met Ala Pro Gly
   1 5
<210> 65
   <211> 5
   <212> PRT
   <213> Homo sapiens
<400> 65
   Val Pro Gly Val Gly
   1 5
<210> 66
   <211> 5
   <212> PRT
   <213> Homo sapiens
<400> 66
   Ile Pro Gly Val Gly
   1 5
<210> 67
   <211> 5
   <212> PRT
   <213> Homo sapiens
<400> 67
   Val Gly Val Pro Gly
   1 5
<210> 68
   <211> 6
   <212> PRT
   <213> Homo sapiens
<400> 68
   Ala Gly Ala Ile Pro Gly
   1               5
<210> 69
   <211> 4
   <212> PRT
   <213> Homo sapiens
<400> 69
   Val Pro Gly Val
   1
<210> 70
   <211> 6
   <212> PRT
   <213> Homo sapiens
<400> 70
   Leu Gly Ile Thr Pro Gly
   1               5
<210> 71
   <211> 4
   <212> PRT
   <213> Homo sapiens
<400> 71
   Gly Asp Asn Pro
   1
<210> 72
   <211> 4
   <212> PRT
   <213> Homo sapiens
<400> 72
   Gly Ala Ile Pro
   1
<210> 73
   <211> 4
   <212> PRT
   <213> Homo sapiens
<400> 73
   Gly Lys Val Pro
   1
<210> 74
   <211> 4
   <212> PRT
   <213> Homo sapiens
<400> 74
   Gly Val Gln Tyr
   1
<210> 75
   <211> 4
   <212> PRT
   <213> Homo sapiens
<400> 75
   Gly Val Leu Pro
   1
<210> 76
   <211> 4
   <212> PRT
   <213> Homo sapiens
<400> 76
   Gly Val Gly Pro
   1
<210> 77
   <211> 4
   <212> PRT
   <213> Homo sapiens
<400> 77
   Gly Phe Gly Pro
   1
<210> 78
   <211> 4
   <212> PRT
   <213> Homo sapiens
<400> 78
   Gly Gly Ile Pro
   1
<210> 79
   <211> 4
   <212> PRT
   <213> Homo sapiens
<400> 79
   Gly Val Ala Pro
   1
<210> 80
   <211> 4
   <212> PRT
   <213> Homo sapiens
<400> 80
   Gly Ile Gly Pro
   1
<210> 81
   <211> 5
   <212> PRT
   <213> Homo sapiens
<400> 81
   Gly Gly Ile Pro Pro
   1 5
<210> 82
   <211> 4
   <212> PRT
   <213> Homo sapiens
<400> 82
   Gly Gln Phe Pro
   1
<210> 83
   <211> 4
   <212> PRT
   <213> Homo sapiens
<400> 83
   Gly Leu Ser Pro
   1
<210> 84
   <211> 4
   <212> PRT
   <213> Homo sapiens
<400> 84
   Gly Pro Gln Pro
   1
<210> 85
   <211> 5
   <212> PRT
   <213> Homo sapiens
<400> 85
   Gly Gly Pro Gln Pro
   1 5
<210> 86
   <211> 5
   <212> PRT
   <213> Homo sapiens
<400> 86
   Gly Pro Gln Pro Gly
   1 5
<210> 87
   <211> 6
   <212> PRT
   <213> Homo sapiens
<400> 87
   Gly Gly Pro Gln Pro Gly
   1 5
<210> 88
   <211> 4
   <212> PRT
   <213> Homo sapiens
<400> 88
   Gly Ile Pro Pro
   1
<210> 89
   <211> 5
   <212> PRT
   <213> Homo sapiens
<400> 89
   Gly Ile Pro Pro Ala
   1               5
<210> 90
   <211> 6
   <212> PRT
   <213> Homo sapiens
<400> 90
   Gly Gly Ile Pro Pro Ala
   1               5
<210> 91
   <211> 58
   <212> PRT
   <213> Homo sapiens
<400> 91
   Gly Gly Tyr Pro Gly Ala Ser Tyr Pro Gly Ala Tyr Pro Gly Gln Ala
   1               5                   10                  15
   Pro Pro Gly Ala Tyr Pro Gly Gln Ala Pro Pro Gly Ala Tyr Pro Gly
               20                  25                  30
   Ala Pro Gly Ala Tyr Pro Gly Ala Pro Ala Pro Gly Val Tyr Pro Gly
           35                  40                  45
   Pro Pro Ser Gly Pro Gly Ala Tyr Pro Ser
       50                  55
<210> 92
   <211> 9
   <212> PRT
   <213> Homo sapiens
<400> 92
   Gly Gly Tyr Pro Gly Ala Ser Tyr Pro
   1               5
<210> 93
   <211> 9
   <212> PRT
   <213> Homo sapiens
<400> 93
   Gly Ala Tyr Pro Gly Gln Ala Pro Pro
   1               5
<210> 94
   <211> 7
   <212> PRT
   <213> Homo sapiens
<400> 94
   Gly Ala Tyr Pro Gly Gln Ala
   1 5
<210> 95
   <211> 7
   <212> PRT
   <213> Homo sapiens
<400> 95
   Gly Ala Tyr Pro Gly Ala Pro
   1 5
<210> 96
   <211> 5
   <212> PRT
   <213> Homo sapiens
<400> 96
   Gly Ala Tyr Pro Gly
   1 5
<210> 97
   <211> 9
   <212> PRT
   <213> Homo sapiens
<400> 97
   Ala Pro Ala Pro Gly Val Tyr Pro Gly
   1 5
<210> 98
   <211> 5
   <212> PRT
   <213> Homo sapiens
<400> 98
   Gly Ala Tyr Pro Ser
   1 5
<210> 99
   <211> 40
   <212> PRT
   <213> Homo sapiens
<400> 99
   Gln Thr Leu Pro Gly Ser Cys Gly Gln Val Val Gly Ser Pro Ser Ala
   1               5                   10                  15
   Gln Asp Glu Ala Ser Pro Leu Ser Glu Trp Arg Ala Ser Tyr Asn Ser
               20                  25                  30
   Ala Gly Ser Asn Ile Thr Asp Ala
           35                  40
<210> 100
   <211> 32
   <212> PRT
   <213> Homo sapiens
<400> 100
   Leu Pro Gly Ser Cys Gly Gln Val Val Gly Ser Pro Ser Ala Gln Asp
   1               5                   10                  15
   Glu Ala Ser Pro Leu Ser Glu Trp Arg Ala Ser Tyr Asn Ser Ala Gly
               20                  25                  30
<210> 101
   <211> 21
   <212> PRT
   <213> Homo sapiens
<400> 101
   Val Val Gly Ser Pro Ser Ala Gln Asp Glu Ala Ser Pro Leu Ser Glu
   1               5                   10                  15
   Trp Arg Ala Ser Tyr
               20
<210> 102
   <211> 15
   <212> PRT
   <213> Homo sapiens
<400> 102
   Val Val Gly Ser Pro Ser Ala Gln Asp Glu Ala Ser Pro Leu Ser
   1               5                   10                  15
<210> 103
   <211> 10
   <212> PRT
   <213> Homo sapiens
<400> 103
   Pro Ser Ala Gln Asp Glu Ala Ser Pro Leu
   1 5 10
<210> 104
   <211> 10
   <212> PRT
   <213> Homo sapiens
<400> 104
   Ser Pro Ser Ala Gln Asp Glu Ala Ser Pro
   1 5 10
<210> 105
   <211> 6
   <212> PRT
   <213> Homo sapiens
<400> 105
   Ala Gln Asp Glu Ala Ser
   1 5
<210> 106
   <211> 4
   <212> PRT
   <213> Homo sapiens
<400> 106
   Pro Ser Ala Gln
   1
<210> 107
   <211> 4
   <212> PRT
   <213> Homo sapiens
<400> 107
   Ser Ala Gln Asp
   1
<210> 108
   <211> 4
   <212> PRT
   <213> Homo sapiens
<400> 108
   Asp Glu Ala Ser
   1
<210> 109
   <211> 4
   <212> PRT
   <213> Homo sapiens
<400> 109
   Ser Pro Ser Ala
   1
<210> 110
   <211> 15
   <212> PRT
   <213> Artificial Sequence
<220>
   <223> antagonist peptide
<400> 110
   Val Val Gly Gly Thr Glu Ala Gln Arg Asn Ser Trp Pro Leu Gln
   1               5                   10                  15
<210> 111
   <211> 15
   <212> PRT
   <213> Artificial Sequence
<220>
   <223> antagonist peptide
<400> 111
   Val Val Gly Gly Thr Glu Ala Gln Arg Asn Ser Trp Pro Ser Gln
   1               5                   10                  15
<210> 112
   <211> 9
   <212> PRT
   <213> Artificial Sequence
<220>
   <223> antagonist peptide
<400> 112
   Thr Glu Ala Gln Arg Asn Ser Trp Pro
   1               5
<210> 113
   <211> 4
   <212> PRT
   <213> Artificial Sequence
<220>
   <223> antagonist peptide
<400> 113
   Ala Gln Arg Asn
   1
<210> 114
   <211> 15
   <212> PRT
   <213> Artificial Sequence
<220>
   <223> antagonist peptide
<400> 114
   Ile Val Gly Gly Arg Arg Ala Arg Pro His Ala Trp Pro Phe Met
   1               5                   10                  15
<210> 115
   <211> 15
   <212> PRT
   <213> Artificial Sequence
<220>
   <223> antagonist peptide
<400> 115
   Val Val Gly Gly Glu Asp Ala Lys Pro Gly Gln Phe Pro Trp Gln
   1               5                   10                  15
<210> 116
   <211> 15
   <212> PRT
   <213> Artificial Sequence
<220>
   <223> antagonist peptide
<400> 116
   Val Val Gly Gly Arg Val Ala Gln Pro Asn Ser Trp Pro Trp Gln
   1               5                   10                  15
<210> 117
   <211> 8
   <212> PRT
   <213> Artificial Sequence
<220>
   <223> antagonist peptide
<400> 117
   Arg Val Ala Gln Pro Asn Ser Trp
   1 5
<210> 118
   <211> 15
   <212> PRT
   <213> Artificial Sequence
<220>
   <223> antagonist peptide
<400> 118
   Val Val Gly Gly Ala Glu Ala Arg Arg Asn Ser Trp Pro Ser Gln
   1 5 10 15
<210> 119
   <211> 8
   <212> PRT
   <213> Artificial Sequence
<220>
   <223> antagonist peptide
<400> 119
   Ala Glu Ala Arg Arg Asn Ser Trp
   1 5
<210> 120
   <211> 15
   <212> PRT
   <213> Artificial Sequence
<220>
   <223> antagonist peptide
<400> 120
   Val Val Gly Gly Gln Glu Ala Thr Pro Asn Thr Trp Pro Trp Gln
   1 5 10 15
<210> 121
   <211> 8
   <212> PRT
   <213> Artificial Sequence
<220>
   <223> antagonist peptide
<400> 121
   Gln Glu Ala Thr Pro Asn Thr Trp
   1 5
<210> 122
   <211> 15
   <212> PRT
   <213> Artificial Sequence
<220>
   <223> antagonist peptide
<400> 122
   Val Val Gly Gly Glu Glu Ala Arg Pro Asn Ser Trp Pro Trp Gln
   1 5 10 15
<210> 123
   <211> 8
   <212> PRT
   <213> Artificial Sequence
<220>
   <223> antagonist peptide
<400> 123
   Glu Glu Ala Arg Pro Asn Ser Trp
   1 5
<210> 124
   <211> 15
   <212> PRT
   <213> Artificial Sequence
<220>
   <223> antagonist
<400> 124
   Val Val Gly Gly Thr Glu Ala Gly Arg Asn Ser Trp Pro Ser Gln
   1 5 10 15
<210> 125
   <211> 9
   <212> PRT
   <213> Artificial Sequence
<220>
   <223> antagonist
<400> 125
   Thr Glu Ala Gly Arg Asn Ser Trp Pro
   1 5
<210> 126
   <211> 15
   <212> PRT
   <213> Artificial Sequence
<220>
   <223> antagonist peptide
<400> 126
   Glu Asp Tyr Arg Pro Ser Gln Gln Asp Glu Cys Ser Pro Arg Glu
   1 5 10 15
<210> 127
   <211> 9
   <212> PRT
   <213> Artificial Sequence
<220>
   <223> antagonist peptide
<400> 127
   Pro Ser Gln Gln Asp Glu Cys Ser Pro
   1 5
<210> 128
   <211> 5
   <212> PRT
   <213> Artificial Sequence
<220>
   <223> antagonist peptide
<400> 128
   Gln Gln Asp Glu Cys
   1 5
<210> 129
   <211> 8
   <212> PRT
   <213> Homo sapiens
<400> 129
   Leu Ala Leu Glu Gly Ser Leu Gln
   1 5
<210> 130
   <211> 4
   <212> PRT
   <213> Homo sapiens
<400> 130
   Ala Gly Gly Pro
   1
<210> 131
   <211> 14
   <212> PRT
   <213> Homo sapiens
<400> 131
   Val Val Gly Ser Pro Ser Ala Gln Asp Glu Ala Ser Pro Leu
   1 5 10
<210> 132
<400> 132
   000
<210> 133
   <211> 7
   <212> PRT
   <213> Homo sapiens
<400> 133
   Gly Gly Gly Pro Gly Ala Gly
   1 5
<210> 134
   <211> 6
   <212> PRT
   <213> Rattus norvegicus
<400> 134
   Leu Gly Gly Gly Pro Glu
   1 5
<210> 135
   <211> 6
   <212> PRT
   <213> Sus scrofa
<400> 135
   Leu Gly Gly Gly Leu Gly
   1 5
<210> 136
   <211> 31
   <212> PRT
   <213> Artificial Sequence
<220>
   <223> retro C-Peptide
<400> 136
   Gln Leu Ser Gly Glu Leu Ala Leu Pro Gln Leu Ser Gly Ala Gly Pro
   1               5                   10                  15
   Gly Gly Gly Leu Glu Val Gln Gly Val Gln Leu Asp Glu Ala Glu
               20                  25                  30
<210> 137
   <211> 6
   <212> PRT
   <213> Homo sapiens
<400> 137
   Leu Gly Thr Ile Pro Gly
   1               5
<210> 138
   <211> 6
   <212> PRT
   <213> Artificial Sequence
<220>
   <223> scrambled peptide
<400> 138
   Gly Val Ala Pro Gly Val
   1               5
<210> 139
   <211> 6
   <212> PRT
   <213> Artificial Sequence
<220>
   <223> scrambled peptide
<400> 139
   Val Ala Pro Gly Val Gly
   1               5
<210> 140
   <211> 6
   <212> PRT
   <213> Artificial Sequence
<220>
   <223> scrambled peptide
<400> 140
   Ala Pro Gly Val Gly Val
   1               5
<210> 141
   <211> 6
   <212> PRT
   <213> Artificial Sequence
<220>
   <223> scrambled peptide
<400> 141
   Pro Gly Val Gly Val Ala
   1               5
<210> 142
   <211> 6
   <212> PRT
   <213> Artificial Sequence
<220>
   <223> scrambled peptide
<400> 142
   Gly Val Gly Val Ala Pro
   1               5
<210> 143
   <211> 10
   <212> PRT
   <213> Homo sapiens
<400> 143
   Val Pro Gly Val Gly Ile Ser Pro Glu Ala
   1               5                   10
<210> 144
   <211> 10
   <212> PRT
   <213> Homo sapiens
<400> 144
   Gly Val Ala Pro Gly Ile Gly Pro Gly Gly
   1               5                   10
<210> 145
   <211> 14
   <212> PRT
   <213> Homo sapiens
<400> 145
   Val Glu Leu Gly Gly Gly Pro Gly Ala Gly Ser Leu Gln Pro
   1               5                   10
<210> 146
   <211> 12
   <212> PRT
   <213> Homo sapiens
<400> 146
   Leu Gly Gly Gly Pro Gly Ala Gly Ser Leu Gln Pro
   1               5                   10
<210> 147
   <211> 9
   <212> PRT
   <213> Homo sapiens
<400> 147
   Leu Gly Gly Gly Pro Gly Ala Gly Ser
   1               5
<210> 148
   <211> 55
   <212> PRT
   <213> Homo sapiens
<400> 148
   Ala Gly Ala Gly Gly Tyr Pro Gly Ala Ser Tyr Pro Gly Ala Tyr Pro
   1               5                   10                  15
   Gly Gln Ala Pro Pro Gly Ala Tyr Pro Gly Gln Ala Pro Pro Gly Ala
               20                  25                  30
   Tyr Pro Gly Ala Pro Gly Ala Tyr Pro Gly Ala Pro Ala Pro Gly Val
           35                  40                  45
   Tyr Pro Gly Pro Pro Ser Gly
       50                  55
<210> 149
   <211> 6
   <212> PRT
   <213> Homo sapiens
<400> 149
   Glu Gly Phe Glu Pro Gly
   1               5
<210> 150
   <211> 31
   <212> PRT
   <213> Homo sapiens
<400> 150
   Glu Ala Glu Asp Leu Gln Val Gly Gln Val Glu Met Gly Gly Gly Pro
   1               5                   10                  15
   Gly Ala Gly Ser Leu Gln Pro Leu Ala Leu Glu Gly Ser Leu Gln
               20                  25                  30
<210> 151
   <211> 31
   <212> PRT
   <213> Homo sapiens
<400> 151
   Glu Ala Glu Asp Leu Gln Val Gly Gln Val Glu Leu Gly Gly Gly Pro
   1               5                   10                  15
   Gly Ala Gly Ser Leu Gln Pro Leu Ala Leu Glu Arg Ser Leu Gln
               20                  25                  30
<210> 152
   <211> 31
   <212> PRT
   <213> Hylobatidae
<400> 152
   Glu Ala Glu Asp Pro Gln Val Gly Gln Val Glu Leu Gly Gly Gly Pro
   1               5                   10                  15
   Gly Ala Gly Ser Leu Gln Pro Leu Ala Leu Glu Gly Ser Leu Gln
               20                  25                  30
<210> 153
   <211> 31
   <212> PRT
   <213> Mus musculus
<400> 153
   Glu Val Glu Asp Pro Gln Val Ala Gln Leu Glu Leu Gly Gly Gly Pro
   1 5 10 15
   Gly Ala Gly Asp Leu Gln Thr Leu Ala Leu Glu Val Ala Gln Gln
   20 25 30
<210> 154
   <211> 31
   <212> PRT
   <213> Rattus norvegicus
<400> 154
   Glu Val Glu Asp Pro Gln Val Ala Gln Leu Glu Leu Gly Gly Gly Pro
   1 5 10 15
   Gly Ala Gly Asp Leu Gln Thr Leu Ala Leu Glu Val Ala Arg Gln
   20 25 30
<210> 155
   <211> 31
   <212> PRT
   <213> Rattus norvegicus
<400> 155
   Glu Val Glu Asp Pro Gln Val Pro Gln Leu Glu Leu Gly Gly Gly Pro
   1 5 10 15
   Glu Ala Gly Asp Leu Gln Thr Leu Ala Leu Glu Val Ala Arg Gln
   20 25 30
<210> 156
   <211> 31
   <212> PRT
   <213> Equus caballus
<400> 156
   Glu Ala Glu Asp Pro Gln Val Gly Gln Glu Glu Leu Gly Gly Gly Pro
   1 5 10 15
   Gly Leu Gly Gly Leu Gln Pro Leu Ala Leu Ala Gly Pro Gln Gln
   20 25 30
<210> 157
   <211> 31
   <212> PRT
   <213> Equus caballus
<400> 157
   Glu Ala Glu Asp Pro Gln Val Gly Glu Val Glu Leu Gly Gly Gly Pro
   1               5                   10                  15
   Gly Leu Gly Gly Leu Gln Pro Leu Ala Leu Ala Gly Pro Gln Gln
               20                  25                  30
<210> 158
   <211> 31
   <212> PRT
   <213> Equus caballus
<400> 158
   Glu Ala Glu Asp Pro Gln Val Gly Gln Val Glu Leu Gly Gly Gly Pro
   1               5                   10                  15
   Gly Leu Gly Gly Leu Gln Pro Leu Ala Leu Ala Gly Pro Gln Gln
               20                  25                  30
<210> 159
   <211> 31
   <212> PRT
   <213> Chinchilla lanigera
<400> 159
   Glu Leu Glu Asp Pro Gln Val Gly Gln Ala Asp Pro Gly Val Val Pro
   1               5                   10                  15
   Glu Ala Gly Arg Leu Gln Pro Leu Ala Leu Glu Met Thr Leu Gln
               20                  25                  30
<210> 160
   <211> 31
   <212> PRT
   <213> Cavia porcellus
<400> 160
   Glu Leu Glu Asp Pro Gln Val Glu Gln Thr Glu Leu Gly Met Gly Leu
   1               5                   10                  15
   Gly Ala Gly Gly Leu Gln Pro Leu Ala Leu Glu Met Ala Leu Gln
               20                  25                  30
<210> 161
   <211> 31
   <212> PRT
   <213> Oryctolagus cuniculus
<400> 161
   Glu Val Glu Glu Leu Gln Val Gly Gln Ala Glu Leu Gly Gly Gly Pro
   1               5                   10                  15
   Gly Ala Gly Gly Leu Gln Pro Ser Ala Leu Glu Leu Ala Leu Gln
               20                  25                  30
<210> 162
   <211> 31
   <212> PRT
   <213> Canis lupus
<400> 162
   Glu Val Glu Asp Leu Gln Val Arg Asp Val Glu Leu Ala Gly Ala Pro
   1               5                   10                  15
   Gly Glu Gly Gly Leu Gln Pro Leu Ala Leu Glu Gly Ala Leu Gln
               20                  25                  30
<210> 163
   <211> 31
   <212> PRT
   <213> Felis catus
<400> 163
   Glu Ala Glu Asp Leu Gln Gly Lys Asp Ala Glu Leu Gly Glu Ala Pro
   1               5                   10                  15
   Gly Ala Gly Gly Leu Gln Pro Ser Ala Leu Glu Ala Pro Leu Gln
               20                  25                  30
<210> 164
   <211> 26
   <212> PRT
   <213> Bos taurus
<400> 164
   Glu Val Glu Gly Pro Gln Val Gly Ala Leu Glu Leu Ala Gly Gly Pro
   1               5                   10                  15
   Gly Ala Gly Gly Leu Glu Gly Pro Pro Gln
               20                  25
<210> 165
   <211> 26
   <212> PRT
   <213> Bos taurus
<220>
   <223> Fleckvieh variant
<400> 165
   Glu Val Glu Gly Pro Gln Val Gly Ala Leu Glu Leu Ala Gly Gly Leu
   1               5                   10                  15
   Gly Ala Gly Gly Leu Glu Gly Pro Pro Gln
               20                  25
<210> 166
   <211> 29
   <212> PRT
   <213> Sus scrofa
<400> 166
   Glu Ala Glu Asn Pro Gln Ala Gly Ala Val Glu Leu Gly Gly Gly Leu
   1               5                   10                  15
   Gly Gly Leu Gln Ala Leu Ala Leu Glu Gly Pro Pro Gln
               20                  25
<210> 167
   <211> 29
   <212> PRT
   <213> Mus musculus
<400> 167
   Glu Val Glu Asp Pro Gln Val Glu Gln Leu Glu Leu Gly Gly Ser Pro
   1               5                   10                  15
   Gly Asp Leu Gln Thr Leu Ala Leu Glu Val Ala Arg Gln
               20                  25
<210> 168
   <211> 20
   <212> PRT
   <213> Homo sapiens
<400> 168
   Pro Val Leu Pro Val Pro Pro Gly Phe Pro Pro Gly Pro Gln Ile Pro
   1               5                   10                  15
   Val Pro Arg Pro
               20
<210> 169
   <211> 11
   <212> PRT
   <213> Homo sapiens
<400> 169
   Thr Cys Glu Glu Gly Phe Glu Pro Gly Pro Met
   1               5                   10
<210> 170
   <211> 41
   <212> PRT
   <213> Homo sapiens
<400> 170
   Gly Leu Val Pro Gly Val Gly Val Ala Pro Gly Val Gly Val Ala Pro
   1               5                   10                  15
   Gly Val Gly Val Ala Pro Gly Val Gly Leu Ala Pro Gly Val Gly Val
               20                  25                  30
   Ala Pro Gly Val Gly Val Ala Pro Gly
           35                  40
<210> 171
   <211> 20
   <212> PRT
   <213> Homo sapiens
<400> 171
   Leu Pro Met Gly Gly Ile Pro Gly Ser Ala Gly Ser Arg Pro Gly Gly
   1               5                   10                  15
   Thr Gly Gly Asn
               20
<210> 172
   <211> 11
   <212> PRT
   <213> Homo sapiens
<400> 172
   Asn Cys Asn Glu Gly Phe Glu Pro Gly Pro Met
   1               5                   10
<210> 173
   <211> 31
   <212> PRT
   <213> Psammomys obesus
<400> 173
   Gly Val Asp Asp Pro Gln Met Pro Gln Leu Glu Leu Gly Gly Ser Pro
   1               5                   10                  15
   Gly Ala Gly Asp Leu Arg Ala Leu Ala Leu Glu Val Ala Arg Gln
               20                  25                  30
<210> 174
   <211> 31
   <212> PRT
   <213> Heterocephalus glaber
<400> 174
   Glu Leu Glu Asn Leu Gln Val Gly Gln Ala Glu Pro Gly Met Gly Leu
   1               5                   10                  15
   Glu Ala Gly Gly Leu Gln Pro Leu Ala Gln Glu Leu Ala Leu Gln
               20                  25                  30
<210> 175
   <211> 18
   <212> PRT
   <213> Homo sapiens
<400> 175
   Val Gly Gln Val Glu Leu Gly Gly Gly Pro Gly Ala Gly Ser Leu Gln
   1               5                   10                  15
   Pro Leu
<210> 176
   <211> 14
   <212> PRT
   <213> Homo sapiens
<400> 176
   Glu Leu Gly Gly Gly Pro Gly Ala Gly Ser Leu Gln Pro Leu
   1               5                   10
<210> 177
   <211> 33
   <212> PRT
   <213> Homo sapiens
<400> 177
   Cys Gly Asn Leu Ser Thr Cys Met Leu Gly Thr Tyr Thr Gln Asp Phe
   1               5                   10                  15
   Asn Lys Phe His Thr Phe Pro Gln Thr Ala Ile Gly Val Gly Ala Pro
               20                  25                  30
   Gly
<210> 178
   <211> 26
   <212> PRT
   <213> Homo sapiens
<400> 178
   Met Leu Gly Thr Tyr Thr Gln Asp Phe Asn Lys Phe His Thr Phe Pro
   1               5                   10                  15
   Gln Thr Ala Ile Gly Val Gly Ala Pro Gly
               20                  25
<210> 179
   <211> 18
   <212> PRT
   <213> Homo sapiens
<400> 179
   Phe Asn Lys Phe His Thr Phe Pro Gln Thr Ala Ile Gly Val Gly Ala
   1               5                   10                  15
   Pro Gly
<210> 180
   <211> 12
   <212> PRT
   <213> Homo sapiens
<400> 180
   Phe Pro Gln Thr Ala Ile Gly Val Gly Ala Pro Gly
   1               5                   10
<210> 181
   <211> 8
   <212> PRT
   <213> Homo sapiens
<400> 181
   Ala Ile Gly Val Gly Ala Pro Gly
   1               5
<210> 182
   <211> 21
   <212> PRT
   <213> Homo sapiens
<400> 182
   Ser His Pro Leu Gly Ser Pro Gly Ser Ala Ser Asp Leu Glu Thr Ser
   1               5                   10                  15
   Gly Leu Gln Glu Gln
               20
<210> 183
   <211> 19
   <212> PRT
   <213> Homo sapiens
<400> 183
   Pro Leu Gly Ser Pro Gly Ser Ala Ser Asp Leu Glu Thr Ser Gly Leu
   1               5                   10                  15
   Gln Glu Gln
<210> 184
   <211> 14
   <212> PRT
   <213> Homo sapiens
<400> 184
   Pro Leu Gly Ser Pro Gly Ser Ala Ser Asp Leu Glu Thr Ser
   1 5 10
<210> 185
   <211> 9
   <212> PRT
   <213> Homo sapiens
<400> 185
   Pro Leu Gly Ser Pro Gly Ser Ala Ser
   1 5
<210> 186
   <211> 6
   <212> PRT
   <213> Homo sapiens
<400> 186
   Pro Leu Gly Ser Pro Gly
   1 5
<210> 187
   <211> 31
   <212> PRT
   <213> Homo sapiens
<400> 187
   Glu Asp Val Ser Ala Gly Glu Asp Cys Gly Pro Leu Pro Glu Gly Gly
   1 5 10 15
   Pro Glu Pro Arg Ser Asp Gly Ala Lys Pro Gly Pro Arg Glu Gly
   20 25 30
<210> 188
   <211> 26
   <212> PRT
   <213> Homo sapiens
<400> 188
   Gly Glu Asp Cys Gly Pro Leu Pro Glu Gly Gly Pro Glu Pro Arg Ser
   1 5 10 15
   Asp Gly Ala Lys Pro Gly Pro Arg Glu Gly
   20 25
<210> 189
   <211> 21
   <212> PRT
   <213> Homo sapiens
<400> 189
   Pro Leu Pro Glu Gly Gly Pro Glu Pro Arg Ser Asp Gly Ala Lys Pro
   1 5 10 15
   Gly Pro Arg Glu Gly
   20
<210> 190
   <211> 17
   <212> PRT
   <213> Homo sapiens
<400> 190
   Pro Leu Pro Glu Gly Gly Pro Glu Pro Arg Ser Asp Gly Ala Lys Pro
   1 5 10 15
   Gly
<210> 191
   <211> 7
   <212> PRT
   <213> Homo sapiens
<400> 191
   Ser Asp Gly Ala Lys Pro Gly
   1 5
<210> 192
   <211> 24
   <212> PRT
   <213> Homo sapiens
<400> 192
   Arg Arg Asn Ala Ser Ser Ala Gly Arg Leu Gln Gly Leu Ala Gly Gly
   1 5 10 15
   Ala Pro Gly Gln Lys Glu Cys Arg
   20
<210> 193
   <211> 16
   <212> PRT
   <213> Homo sapiens
<400> 193
   Arg Leu Gln Gly Leu Ala Gly Gly Ala Pro Gly Gln Lys Glu Cys Arg
   1 5 10 15
<210> 194
   <211> 20
   <212> PRT
   <213> Homo sapiens
<400> 194
   Arg Arg Asn Ala Ser Ser Ala Gly Arg Leu Gln Gly Leu Ala Gly Gly
   1               5                   10                  15
   Ala Pro Gly Gln
               20
<210> 195
   <211> 11
   <212> PRT
   <213> Homo sapiens
<400> 195
   Leu Gln Gly Leu Ala Gly Gly Ala Pro Gly Gln
   1               5                   10
<210> 196
   <211> 6
   <212> PRT
   <213> Homo sapiens
<400> 196
   Ala Gly Gly Ala Pro Gly
   1               5
<210> 197
   <211> 6
   <212> PRT
   <213> Homo sapiens
<400> 197
   Tyr Gly Tyr Gly Pro Gly
   1               5
<210> 198
   <211> 6
   <212> PRT
   <213> Homo sapiens
<400> 198
   Tyr Gly Ala Arg Pro Gly
   1               5
<210> 199
   <211> 6
   <212> PRT
   <213> Homo sapiens
<400> 199
   Phe Gly Ala Val Pro Gly
   1 5
<210> 200
   <211> 6
   <212> PRT
   <213> Homo sapiens
<400> 200
   Gly Val Gly Ala Pro Gly
   1 5
<210> 201
   <211> 6
   <212> PRT
   <213> Homo sapiens
<400> 201
   Asp Gly Ala Lys Pro Gly
   1 5
<210> 202
   <211> 6
   <212> PRT
   <213> Homo sapiens
<400> 202
   Gln Gly Met Leu Pro Gly
   1 5
<210> 203
   <211> 10
   <212> PRT
   <213> Homo sapiens
<400> 203
   Phe Gly Leu Val Pro Gly Val Gly Val Ala
   1 5 10
<210> 204
   <211> 10
   <212> PRT
   <213> Homo sapiens
<400> 204
   Gly Val Ala Pro Gly Ile Gly Pro Gly Gly
   1 5 10
<210> 205
   <211> 28
   <212> PRT
   <213> Homo sapiens
<400> 205
   Pro Pro Gly Ala Tyr Pro Gly Gln Ala Pro Pro Gly Ala Tyr Pro Gly
   1 5 10 15
   Ala Pro Gly Ala Tyr Pro Gly Ala Pro Ala Pro Gly
   20 25
<210> 206
   <211> 10
   <212> PRT
   <213> Homo sapiens
<400> 206
   Thr Cys Glu Glu Gly Phe Glu Pro Gly Pro
   1 5 10
<210> 207
   <211> 10
   <212> PRT
   <213> Homo sapiens
<400> 207
   Asn Pro Leu Gly Thr Ile Pro Gly Gly Asn
   1 5 10
<210> 208
   <211> 35
   <212> PRT
   <213> Homo sapiens
<400> 208
   Arg Arg Glu Ala Glu Asp Leu Gln Val Gly Gln Val Glu Leu Gly Gly
   1 5 10 15
   Gly Pro Gly Ala Gly Ser Leu Gln Pro Leu Ala Leu Glu Gly Ser Leu
   20 25 30
   Gln Lys Arg
   35
<210> 209
   <211> 18
   <212> PRT
   <213> Homo sapiens
<400> 209
   Arg Val Leu Gln Gly Val Leu Pro Ala Leu Pro Gln Val Val Cys Asn
   1               5                   10                  15
   Tyr Arg
<210> 210
   <211> 38
   <212> PRT
   <213> Homo sapiens
<400> 210
   Lys Arg Cys Gly Asn Leu Ser Thr Cys Met Leu Gly Thr Tyr Thr Gln
   1               5                   10                  15
   Asp Phe Asn Lys Phe His Thr Phe Pro Gln Thr Ala Ile Gly Val Gly
               20                  25                  30
   Ala Pro Gly Lys Lys Arg
           35
<210> 211
   <211> 23
   <212> PRT
   <213> Homo sapiens
<400> 211
   Arg Ser His Pro Leu Gly Ser Pro Gly Ser Ala Ser Asp Leu Glu Thr
   1               5                   10                  15
   Ser Gly Leu Gln Glu Gln Arg
               20
<210> 212
   <211> 35
   <212> PRT
   <213> Homo sapiens
<400> 212
   Lys Arg Glu Asp Val Ser Ala Gly Glu Asp Cys Gly Pro Leu Pro Glu
   1               5                   10                  15
   Gly Gly Pro Glu Pro Arg Ser Asp Gly Ala Lys Pro Gly Pro Arg Glu
               20                  25                  30
   Gly Lys Arg
           35
<210> 213
   <211> 17
   <212> PRT
   <213> Homo sapiens
<400> 213
   Arg Ala Ala Pro Leu Gln Gly Met Leu Pro Gly Leu Leu Ala Pro Leu
   1               5                   10                  15
   Arg
<210> 214
   <211> 6
   <212> PRT
   <213> Homo sapiens
<400> 214
   Phe Gly Leu Val Pro Gly
   1               5
<210> 215
   <211> 6
   <212> PRT
   <213> Homo sapiens
<400> 215
   Pro Gly Ile Gly Pro Gly
   1               5
<210> 216
   <211> 42
   <212> PRT
   <213> Homo sapiens
<400> 216
   Val Gly Val Ala Pro Gly Val Gly Val Ala Pro Gly Val Gly Val Ala
   1               5                   10                  15
   Pro Gly Val Gly Leu Ala Pro Gly Val Gly Val Ala Pro Gly Val Gly
               20                  25                  30
   Val Ala Pro Gly Val Gly Val Ala Pro Gly
           35                  40

## Description

### Technical field

The disclosure belongs to the field of human medicine, and belongs to the field of pharmacy, biotechnology and drug development. The disclosure relates to the etiology of metabolic syndrome and provides use of immune-modulatory peptides for treatment of inflammation, insulin resistance, atheromatous disease, arteriosclerosis, atherosclerosis, cardiovascular disease, micro- and macrovascular pathologies in type 1 and type 2 diabetes mellitus, in humans.

### Background

Our all-too-human habit to overeat and the easy availability of everyday food have resulted in a worldwide obesity epidemic with dire consequences to our health. One-and-half (1.5) billion people are overweight (of which 0.5 billion obese), and a great many of those suffer from a chronic inflammatory disease often called metabolic syndrome: the major cause of unhealthy aging and death in high- and middle-income countries. These 1.5 billion people are at increased risk to develop cardiovascular disease (chronic inflammation of the blood vessels [atheromatous disease, arteriosclerosis, atherosclerosis], increased blood pressure [hypertension] and increased abnormal fat levels [dyslipidaemia] in the blood), leading up to heart attack and stroke. At least 30% of these 1.5 billion are at further risk to develop diabetes type 2 (World Health Organization). Others develop too early manifestations of aging such as kidney failure or dementia. Heart failure, as non-fatal and fatal myocardial infarction and peripheral arterial disease (PAD) are the most common initial manifestations of cardiovascular disease in type 2 diabetes, others are transient ischemic attacks (TIA) or ischaemic stroke and stable angina. Living a sedentary life and smoking further increases risks of dying from these conditions. Currently, no satisfying medical understanding (other than excess diet) exists of the causal events leading to the initially mild but ultimately chronic inflammatory disease that underlies these staggering figures. Why this food-intake-induced inflammation occurs and affects so many people is largely unknown and human of much debate.

C-peptide is the linking peptide between the A- and B-chains in the proinsulin molecule. After cleavage in the endoplasmic reticulum of pancreatic islet beta-cells, insulin and a 35-amino acid peptide are generated. The latter is processed to the 31-amino acid peptide, C-peptide, by enzymatic removal of two basic residues on either side of the molecule. C-peptide is co-secreted with insulin in equimolar amounts from the pancreatic islet beta-cells into the portal circulation. Besides its contribution to the folding of the two-chain insulin structure, further biologic activity of C-peptide was questioned for many years after its discovery.

### Human C-peptide is a 31-amino acid peptide having the sequence

SEQ ID NO: 1 (EAEDLQVGQVELGGGPGAGSLQPLALEGSLQ, in the one-letter amino acid code).

C-peptide classically is ascribed a tripartite overall structure, with more conserved N- and C-terminal segments and a more variable mid-sequence, or internal, and hydrophobic midportion. Thus, in the case of human C-peptide the N-terminal segment is often regarded as residues 1-12 (SEQ ID NO: 2 (EAEDLQVGQVEL)), the mid-portion as residues 13-25 (SEQ ID NO: 3 (GGGPGAGSLQPLA)), and the C-terminal segment as residues 25-31 (SEQ ID NO: 4 (LEGSLQ)). The tetrapeptide SEQ ID NO: 5 (EAED) is thought to be required in the process of folding the two-chain insulin structure in the beta cells (Chen at al., J Biochem. 2002 Jun;131(6):855-9). Recently, some studies suggested that the C-terminal pentapeptide (SEQ ID NO: 6 EGSLQ) in human C-peptide and SEQ ID NO: 7 (EVARQ) in rat C-peptide) of C-peptide which shows a well-defined secondary structure may induce intracellular Ca2+ increases in human renal tubular cells (Shafqat et al., Cell Mol Life Sci. 2002 Jul;59(7):1185-9.).

C-peptide is produced in equal amounts to insulin and is the best additional measure of endogenous insulin secretion in patients with diabetes. Measurement of insulin secretion using C-peptide is considered helpful in clinical practice: differences in insulin secretion are fundamental to the different treatment requirements of Type 1 and Type 2 diabetes. Jones and Hattersley (Diabet Med. 2013 Jul;30(7):803-17) review the use of C-peptide measurement in the clinical management of patients with diabetes, including the interpretation and choice of C-peptide test and its use to assist diabetes classification and choice of treatment, and recommendations for where C-peptide should be used, choice of test and interpretation of results. As the relationships between C-peptide levels and metabolic control and chronic complications are poorly known in type 2 diabetes, due to the slow decline of beta-cell function, Bo et al., (Acta Diabetol. 2000;37(3):125-9) evaluated these associations in a cohort of type 2 diabetic patients. Biological effects of C-peptide are thought to be mediated by interaction with insulin or via specific or nonspecific membrane interaction. Some studies in the art support the theory of specific interactions with a yet to be indentified GPCR. However, the D-enantiomer of C-peptide has the same biological activity as the L-enantiomer; (Ido et al., Science, 1997, 277(5325):563-6), thus finding reverse (retro) and all-D-amino acid (enantio) C-peptides equipotent to native C-peptide, IDO et al. conclude the activity of SEQ ID NO: 8 (GGGPGAG) to be not mediated by a receptor, thereby teaching away from a receptor for C-peptide, which has suggested to those in the art that other, receptor-independent, interactions are important for function of C-peptde. Formation of cation-selective channels in lipid bilayers has also led to suggestions of a more nonspecific interaction. Thus, a receptor for C-peptide has remained elusive.

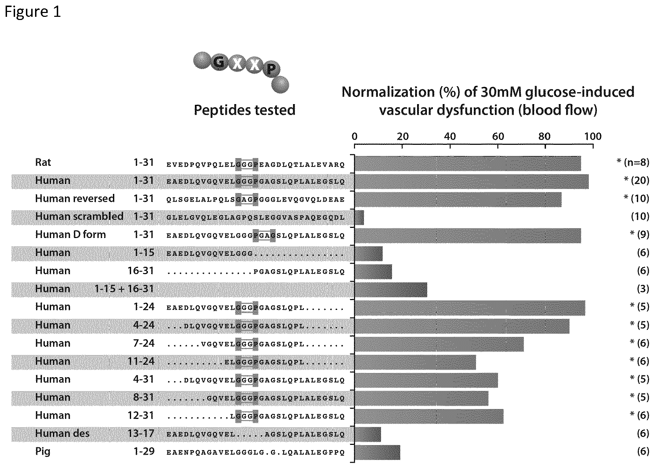

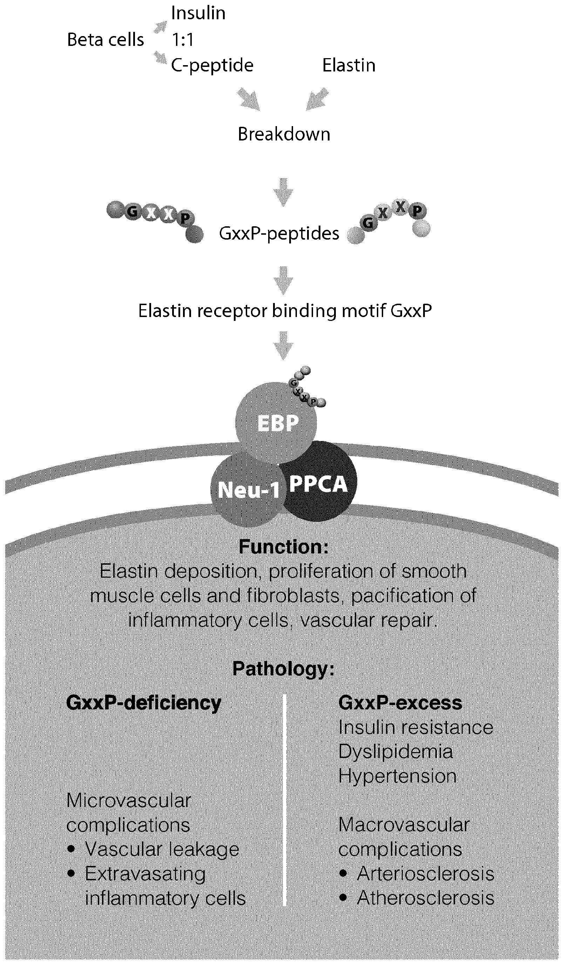

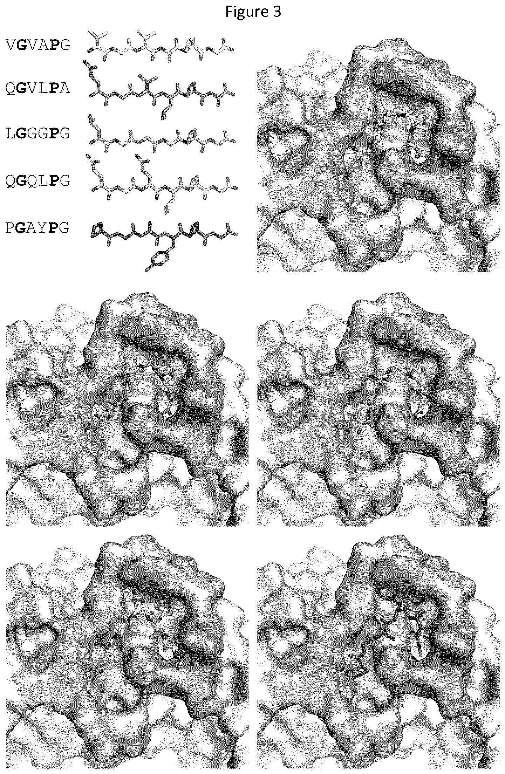

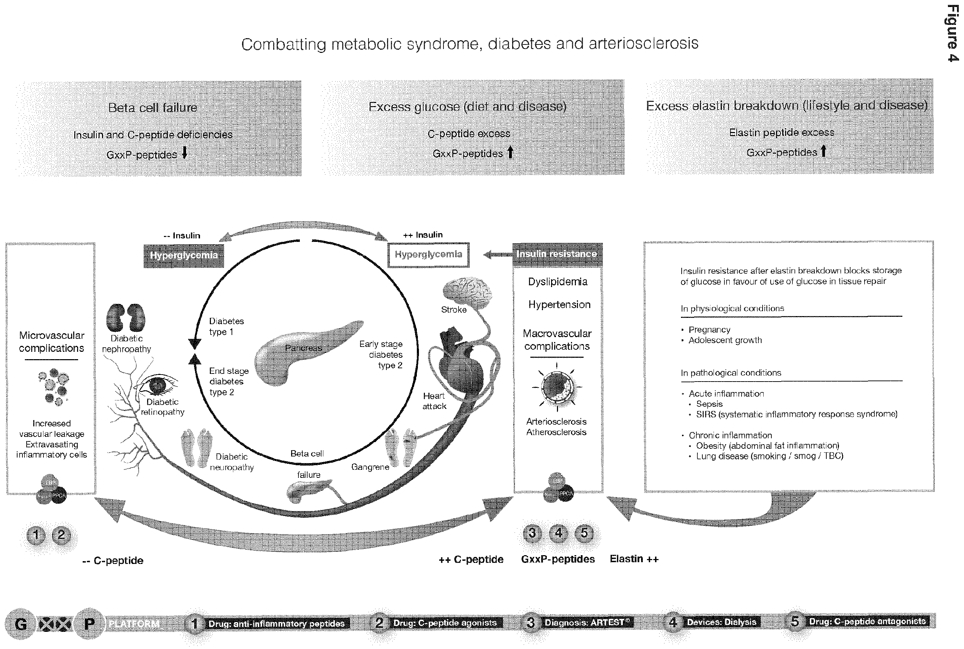

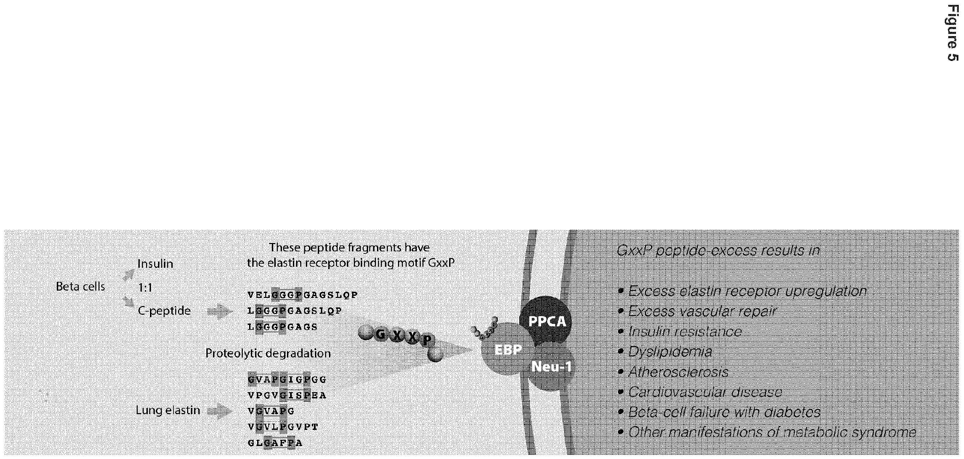

Ido et al. (Science, 1997 Jul 25;277(5325):563-6; and figure 1 in this application) show human C-peptide fragments with hydrophobic midportion SEQ ID NO: 8 (GGGPGAG) to normalize glucose-induced vascular dysfunction in rat granular tissue, they however, have not provided testing of C-peptide fragments in combination with treatment of those rats with insulin, and teach away from receptor-mediated activity of fragments having said hydrophobic midportion. In US20020107175 a C-peptide fragment SEQ ID NO: 9 (ELGGGPGAG) and some of its smaller fragments stimulate Na.sup.+K.sup.+ATPase activity of rat renal tubule cells. US20060234914 and 20070082842 list N-terminal- and/or C-terminal-C-peptide fragments that comprise at least one glutamine (in three letter code Glu; in one-letter code E) to provide biological activity not related to the activity of above discussed hydrophobic midportion SEQ ID NO: 8 (GGGPGAG), which midportion sequence is not found in any of the fragments listed in US20060234914 nor 20070082842.

Type 1 diabetes is generally characterized by insulin and C-peptide deficiency, due to an autoimmune destruction of the pancreatic islet beta-cells. The patients are therefore dependent on exogenous insulin to sustain life. Several factors may be of importance for the pathogenesis of the disease, e.g., genetic background, environmental factors, and an aggressive autoimmune reaction following a temporary infection (Akerblom H K et al.: Annual Medicine 29(5): 383-385, (1997)). Currently insulin-requiring patients are provided with exogenous insulin which has been separated from the C-peptide, and thus do not receive exogenous C-peptide therapy. By contrast, most type 2 diabetic subjects initially still produce both insulin and C-peptide endogenously, but are generally characterized by insulin resistance in skeletal muscle, adipose tissue, and liver, among other tissues.

Many type 1 and end-phase type 2 diabetic patients (that do not longer produce insulin and C-peptide) and other insulin-requiring patients eventually develop and suffer from a constellation of long-term vascular complications of diabetes that in many cases are more severe and widespread than in early phase type 2 diabetes (wherein insulin and C-peptide are stil produced but the patient is resistant to insulin. For example, microvascular complications involving the retina, kidneys, and nerves are a major cause of morbidity and mortality in patients with type 1 diabetes or end-phase type 2 diabetes but are generally considered not prominent in patients that are resistant to insulin. There is increasing support for the concept that C-peptide deficiency may play a role in the development of the long-term complications of insulin-requiring diabetic patients. Additionally, in vivo as well as in vitro studies in diabetic human models and in patients with type 1 diabetes demonstrate that C-peptide possesses hormonal activity (Wahren J et al.: American Journal of Physiology 278: E759-E768, (2000); Wahren J et al.: In International Textbook of Diabetes Mellitus Ferranninni E, Zimmet P, De Fronzo R A, Keen H, Eds. John Wiley & Sons, (2004), p. 165-182).

US 2007/0082842 discloses the therapeutic use of different C-peptides and fragments (some of which comprise the motif GxxP or GxxPOG) thereof for the treatment of diabetes and microvascular complications thereof. WO 2005/118638 discloses degradation resistant analogues of C-peptides and fragments thereof for the treatment of diabetes and diabetic complications. Qu'aty et al (2015, J. Dermatol. Sci. Vol. 80, No.3, pp.175-185) discloses the use of synthetic peptides mimicking the elastin receptor binding motif for regeneration of injured or aged human skin. Robinet et al (2004, J. Cell Sci. Vol. 118, No. 2, pp. 343-356) discloses synthetic elastin-derived peptides (i.e. VGVAPG) for use in in vitro tubulogenesis and wound healing assays. Kawecki et al (2014, Arteriosclerosis, Thr. Vasc. Biol. Vol. 34, No. 12 pp. 2570-2578) discloses the role of elastin-derived peptides as regulators of thrombosis. Blaise et al (2013, Diabetes Vol. 62, No. 11, pp. 3807-3816) discloses the role of elastin-derived peptides as regulators of insulin resistance in mice. Blanchevoye et al (2013, J. Biol. Chem. Vol. 288, No. 2, pp. 1317-1328) shows structural data describing the interaction between the elastin peptide VGVAPG and the human elastin receptor. However, none of these documents teaches or suggests the use of the peptides in any therapy for inflammatory disease. Maurice et al (2013, Trends Cardiovasc Med. 23(6):211-21) mentions the use of peptide V14 (VVGSPSAQDEAPL) as an antagonist that affects the binding of elastin-derived peptides (or of the peptide VGVAPG) to the elastin-receptor in mice.

### The invention

The invention provides an isolated or synthetic peptide for use in treatment of human inflammation, , wherein the peptide has at least one human elastin receptor binding motif GxxPG and has at least one amino acid Q, wherein G represents the one-letter code for the amino acid glycine, P for the amino acid proline, Q for the amino acid glutamine and x for any amino acid, the peptide consisting of 5-30 amino acids. Typically preferred peptides provided herein are selected from the group peptides listed under SEQ ID NOs: 10, 11, 12, 13, 17, 18, 19, 20, 14, 15, 16, 21, 25, 175, 3, 22, 23, 26, 176, 24, 27, 28, 29, 43, 93, and 94, and retro-inverso variant peptides derived from peptides listed under SEQ ID NOs: 10, 11, 12, 13, 17, 18, 19, 20, 14, 15, 16, 21, 25, 175, 3, 22, 23, 26, 176, 24, 27, 28, 29, 43, 93, and 94. Note: Retro-inverse peptides are composed of D-amino acids assembled in a reverse order from that of the parent L-sequence, thus maintaining the overall topology of the native sequence. No stereoisomers of glycine exist, here (and in retro-inverso peptides bearing for example retro-inverso GxxP or xGxP motifs) G is not, whereas other amino acids, such as L, P and A are, instrumental to the all-D-amino acid character of the retro-inverso peptide herein provided. The invention also provides synthetic peptides wherein the human elastin receptor binding motif GxxPG motif has been repeated at least twice, preferably thrice, optionally said repeats are separated by a linker, such a linker may comprise one or more amino acids, such as one or more amino acids selected from the group of glycine, alanine, leucine, valine, isoleucine or glutamine. In a preferred embodiment, the invention provides a peptide capable of combining with a human elastin receptor on a cell and initiating the same physiological activity typically produced by the binding of human C-peptide to the human elastin receptor. The invention also provides such an isolated or synthetic peptide for use in treatment of human disease, such as in treatment of human inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one human elastin receptor binding motif GxxPG and has at least one amino acid Q, wherein G represents the one-letter code for the amino acid glycine, P for the amino acid proline, Q for the amino acid glutamine and x for any amino acid, the peptide consisting of 5-20 amino acids. Typically preferred peptides provided herein are selected from the group peptides listed under SEQ ID NOs: 14, 15, 16, 21, 25, 175, 3, 22, 23, 26, 176, 24, 27, 28, 29, 43, 93, and 94, and retro-inverso variant peptides derived from peptides listed under SEQ ID NOs: 14, 15, 16, 21, 25, 175, 3, 22, 23, 26, 176, 24, 27, 28, 29, 43, 93, and 94.

In a preferred embodiment, the invention also provides a peptide capable of combining with a human elastin receptor on a cell and initiating the same physiological activity typically produced by the binding of human C-peptide to the human elastin receptor. The invention also provides such an isolated or synthetic peptide for use in treatment of human disease, such as in treatment of human inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one human elastin receptor binding motif GxxPG and has at least one amino acid Q, wherein G represents the one-letter code for the amino acid glycine, P for the amino acid proline, Qfor the amino acid glutamine and x for any amino acid, the peptide consisting of 5-15 amino acids. Typically preferred peptides provided herein are selected from the group peptides listed under SEQ ID NOs: 3, 22, 23, 26, 176, 24, 27, 28, 29, 43, 93, and 94, and retro-inverso variant peptides derived from peptides listed under SEQ ID NOs: 3, 22, 23, 26, 176, 24, 27, 28, 29, 43, 93, and 94.

In a preferred embodiment, the invention also provides a peptide capable of combining with a human elastin receptor on a cell and initiating the same physiological activity typically produced by the binding of human C-peptide to the human elastin receptor. The invention also provides such an isolated or synthetic peptide for use in treatment of human disease, such as in treatment of human inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one human elastin receptor binding motif GxxPG and has at least one amino acid Q, wherein G represents the one-letter code for the amino acid glycine, P for the amino acid proline, Q for the amino acid glutamine and x for any amino acid, the peptide consisting of 5-12 amino acids. Typically preferred peptides provided herein are selected from the group peptides listed under SEQ ID NOs: 24, 27, 28, 29, 43, 93, and 94, and retro-inverso variant peptides derived from peptides listed under SEQ ID NOs: 24, 27, 28, 29, 43, 93, and 94.

In a preferred embodiment, the invention provides a peptide capable of combining with a human elastin receptor on a cell and initiating the same physiological activity typically produced by the binding of human C-peptide to the human elastin receptor. The invention also provides such an isolated or synthetic peptide for use in treatment of human disease, such as in treatment of human inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one human elastin receptor binding motif GxxPG and has at least one amino acid Q, wherein G represents the one-letter code for the amino acid glycine, P for the amino acid proline, Q for the amino acid glutamine and x for any amino acid, the peptide consisting of 5-9 amino acids. Typically preferred peptides provided herein are selected from the group peptides listed under SEQ ID NOs: 29, 43, 93, and 94, and retro-inverso variant peptides derived from peptides listed under SEQ ID NOs: 29, 43, 93, and 94.

The invention relates and is limited to the use of peptide agonists and/or peptide antagonists of human C-peptide's interaction with the human elastin receptor for treatment of human disease.

A first field of the invention, named Drug: anti-inflammatory peptides, shall mean any peptide for use as a drug to counteract (control, reduce or treat) human inflammatory disease or to any peptide component of a drug to counteract human inflammatory disease or to any peptide used in the preparation of a drug to counteract human inflammatory disease (counteract in this descripton also generally indentified as treat or use in treatment), wherein the peptide has at least one human elastin receptor binding motif GxxP, or its functionally equivalent xGxP, has at least one amino acid Q, and wherein G represents the one-letter code for the amino acid glycine, P for the amino acid proline, Q for the amino acid glutamine and x for any amino acid. Preferably said peptide of this first field has at least one human elastin receptor binding motif xGxxPG. Such peptides are useful in the treatment of inflammatory conditions, such as acute kidney injury, also in acute systemic inflammatory conditions such as for example sepsis or systemic inflammatory response syndrome (SIRS), leading to vascular damage and often aggravated by (multiple organ) organ failure, or inflammatory conditions with diabetes, when given with an anti-diabetic composition such as insulin.

Herein a peptide or peptide fragment having a PG-domain is particulary defined as a peptide having at least one xGxP, GxxP, GxxPG or xGxPG motif, G being Glycine, P being Proline, x being any amino acid, the amino acid following P preferably allowing for a type VIII-beta turn, a condition which is met when P is C-terminally followed by a G. The invention provides a method for preventing or treating disease comprising providing a human with a peptide capable of agonising an elastin receptor. Such peptides as herein provided are preferably selected from the group of fragments of C-peptide and functional equivalents thereof.

In a first embodiment, the invention provides a peptide for use in the treatment of human inflammation, preferably of a human subject in need thereof, in particularly in a human subject having been diagnosed as suffering from type 1 diabetes, most preferably when said subject is also treated with insulin, wherein said peptide is selected from the group of, preferably isolated and/or synthetic, preferably non-peggylated, C-peptide fragments human 1-24 SEQ ID NO: 17 (EAEDLQVGQVELGGGPGAGSLQPL), human 4-24 SEQ ID NO: 20 (DLQVGQVELGGGPGAGSLQPL), human 7-24 SEQ ID NO: 175 (VGQVELGGGPGAGSLQPL), human 11-24 SEQ ID NO: 176 (ELGGGPGAGSLQPL), human 4-31 SEQ ID NO: 10 (DLQVGQVELGGGPGAGSLQPLALEGSLQ), human 8-31 SEQ ID NO: 13 (GQVELGGGPGAGSLQPLALEGSLQ) and human 12-31 SEQ ID NO: 14 (LGGGPGAGSLQPLALEGSLQ), as listed in figure 1 in this application, said peptide showing the GxxP motif SEQ ID NO: 38 (GGGP), and a significant normalization (%) of 30 mM glucose-induced vascular dysfunction in rats.

Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 10 (DLQVGQVELGGGPGAGSLQPLALEGSLQ) as derivable from the human C-peptide sequence. Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 11 (LQVGQVELGGGPGAGSLQPLALEGSLQ) as obtainable from the human C-peptide sequence. Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, most preferably when said subject is also treated with insulin, as provided herein is the synthetic and isolated peptide SEQ ID NO: 12 (VGQVELGGGPGAGSLQPLALEGSLQ) as derivable from the human C-peptide sequence. Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 13 (GQVELGGGPGAGSLQPLALEGSLQ) as derivable from the human C-peptide sequence. Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 14 (LGGGPGAGSLQPLALEGSLQ) as derivable from the human C-peptide sequence. Also, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 15 (VGQVELGGGPGAGSLQPLAL) as derivable from the human C-peptide sequence. Also, a peptide with motif SEQ ID NO: 38 (GGGP)- for use in the treatment of human inflammation, in particularly in a human subject having been diagnosed as suffering from type 1 diabetes, most preferably when said subject is also treated with insulin, as provided herein is the synthetic and isolated peptide SEQ ID NO: 16 (EVGQVELGGGPGAGSLQPL) as derivable from the human C-peptide sequence. In another embodiment, a peptide with motif SEQ ID NO: 38 (GGGP)-listed in figure 1 herein, is provided for use in the treatment of human inflammation, in particularly in a human subject having been diagnosed as suffering from type 1 diabetes, most preferably when said subject is also treated with insulin, as provided herein is the synthetic and isolated peptide SEQ ID NO: 17 (EAEDLQVGQVELGGGPGAGSLQPLAL) as derivable from the human C-peptide sequence. In another embodiment, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 18 (EAEDLQVGQVELGGGPGAGSLQPL) as derivable from the human C-peptide sequence. In another embodiment a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 19 (LQVGQVELGGGPGAGSLQPLAL) as derivable from the human C-peptide sequence. In another embodiment, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 20 (DLQVGQVELGGGPGAGSLQPL) as derivable from the human C-peptide sequence. In another embodiment, a peptide with motif SEQ ID NO: 38 (GGGP)-listed in figure 1 herein, is provided for use in the treatment of human inflammation, in particularly in a human subject having been diagnosed as suffering from type 1 diabetes, most preferably when said subject is also treated with insulin, as provided herein is the synthetic and isolated peptide SEQ ID NO: 21 (LQVGQVELGGGPGAGSLQPL) as derivable from the human C-peptide sequence. In another embodiment, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 22 (LGGGPGAGSLQPL) as derivable from the human C-peptide sequence. In another embodiment, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 23 (VGQVELGGGPGAGSL) as derivable from the human C-peptide sequence. In another embodiment, a peptide with motif SEQ ID NO: 38 (GGGP)-listed in figure 1 herein, for use in the treatment of human inflammation, in particularly in a human subject having been diagnosed as suffering from type 1 diabetes, most preferably when said subject is also treated with insulin, as provided herein is the synthetic and isolated peptide SEQ ID NO: 24 (GGGPGAGSLQ) as derivable from the human C-peptide sequence.

The invention also provides an isolated and/or synthetic, preferably non-peggylated, peptide identified herein as a regulatory model element peptide or fragment thereof that it is identified herein in specific regulatory elements modulating inflammation and tissue repair. The invention provides an isolated and/or synthetic peptide wherein said regulatory model element peptide or fragment preferably carries a xGxxPG or xxGxPG motif and preferably can be derived for example from proteins identified in Table 3 herein. In a preferred embodiment, the model element is recognized while it is flanked by at least one N-terminal and at least one C-terminal basic amino acid residue R (arginine) or K (lysine). Smaller fragments from within the element not carrying the two flanking basic residues are also usefull in modulating inflammation and tissue repair. In one embodiment, a regulatory model element peptide fragment for use in the treatment of human inflammation as provided herein is the synthetic and isolated peptide SEQ ID NO: 25 (FRAAPLQGMLPGLLAPLRT) as derivable from the human COL6A3 sequence (in Uniprot database COL6A3 is known under identifier P12111 and the SEQ ID NO: 25 (FRAAPLQGMLPGLLAPLRT) sequence is found in the sequence in isoform 1 from at around position 605-626). It is herein provided that said peptide is useful in the treatment of human inflammation and/or tissue repair, as are fragments thereof. In another embodiment, a regulatory model element peptide fragment for use in the treatment of human inflammation and/or tissue repair as provided herein is the synthetic and isolated peptide SEQ ID NO: 26 (AAPLQGMLPGLLAPL) as derivable from the human COL6A3 sequence. In another embodiment, a regulatory model element peptide fragment for use in the treatment of human inflammation and/or tissue repair as provided herein is the synthetic and isolated peptide SEQ ID NO: 27 (LQGMLPGLLAPL) as derivable from the human COL6A3 sequence. In another embodiment, a regulatory model element peptide fragment for use in the treatment of human inflammation and/or tissue repair as provided herein is the synthetic and isolated peptide SEQ ID NO: 28 (LQGMLPGLLA) as derivable from the human COL6A3 sequence. In another embodiment, a regulatory model element peptide fragment for use in the treatment of human inflammation and/or tissue repair as provided herein is the synthetic and isolated peptide SEQ ID NO: 29 (LQGMLPG) as derivable from the human COL6A3 sequence.

In another embodiment, a regulatory model element peptide fragment for use in the treatment of human inflammation and/or tissue repair as provided herein is the synthetic and isolated peptide SEQ ID NO: 178 (MLGTYTQDFNKFHTFPQTAIGVGAPG) as derivable from the human procalcitonin sequence.

In another embodiment, a regulatory model element peptide fragment for use in the treatment of human inflammation and/or tissue repair as provided herein is the synthetic and isolated peptide SEQ ID NO: 179 (FNKFHTFPQTAIGVGAPG) as derivable from the human procalcitonin sequence. In another embodiment, a regulatory model element peptide fragment for use in the treatment of human inflammation and/or tissue repair as provided herein is the synthetic and isolated peptide SEQ ID NO: 180 (FPQTAIGVGAPG) as derivable from the human procalcitonin sequence

In another embodiment, a regulatory model element peptide fragment for use in the treatment of human inflammation and/or tissue repair as provided herein is the synthetic and isolated peptide SEQ ID NO: 182 (SHPLGSPGSASDLETSGLQEQ) as derivable from the human NTproBNP sequence. In another embodiment, a regulatory model element peptide fragment for use in the treatment of human inflammation and/or tissue repair as provided herein is the synthetic and isolated peptide SEQ ID NO: 183 (PLGSPGSASDLETSGLQEQ) as derivable from the human NTproBNP sequence. In another embodiment, a regulatory model element peptide fragment for use in the treatment of human inflammation and/or tissue repair as provided herein is the synthetic and isolated peptide SEQ ID NO: 193 (RLQGLAGGAPGQKECR) as derivable from the human pyrin sequence. In another embodiment, a regulatory model element peptide fragment for use in the treatment of human inflammation and/or tissue repair as provided herein is the synthetic and isolated peptide SEQ ID NO: 194 (RRNASSAGRLQGLAGGAPGQ) as derivable from the human pyrin (marenostrin) sequence.

In another embodiment, a regulatory model element peptide fragment for use in the treatment of human inflammation and/or tissue repair as provided herein is the synthetic and isolated peptide SEQ ID NO: 195 (LQGLAGGAPGQ) as derivable from the human pyrin sequence. The invention also discloses use in humans of a peptide consisting of 4-40 amino acids, preferably of 4-20, more preferably of 4-15, more preferably 4-12, most preferably of 4-9 amino acids, the peptide comprising at least one PG-domain, preferably with a xGxP or GxxP, GxxPG or xGxPG motif.

The invention also provides a method for preventing or treating disease of a human comprising providing said human with a peptide capable of agonising an elastin receptor of said human.

The invention shows that the so-called inflammation in metabolic syndrome is augmented by an hitherto overlooked lock- and-key activation of the elastin receptor, a protein involved in vascular (blood vessel) elastin repair, with the C-peptide, a small protein that is produced in a 1:1 ratio alongside with widely known insulin. The human elastin receptor is the lock that is activated by a key motif of amino acids (GxxP) found in C-peptide and in breakdown products (GxxP-fragments) thereof. Until now, no one has ever discovered this lock-and-key interaction between the two, now providing novel inroads in development of novel peptides for treatment of metabolic syndrome, exploiting the finding that not only the normal keys of the elastin receptor (elastin peptides), but also the C-peptide, a peptide we produce together with insulin every time glucose rises in our blood after a meal, interacts in a lock-and-key mode with the elastin receptor. In summary, the invention provides the insight that excess food intake directly switches C-peptide on as the key unlocking metabolic syndrome. Everyday overeating results in everyday increased C-peptide levels (and GxxP motif containing break-down fragments thereof) in the blood. As the elastin receptor is mainly found on cells that produce elastin and on cells that repair our blood vessels (together called vascular cells), everyday GxxP lock-and-key activation of the elastin receptor by excess C-peptide and its fragments results in everyday blood vessel damage done. As 30% of the walls of our blood-vessels are made up of elastin and inflammatory cells continuously repair damage done to blood vessels, disturbing elastin repair and provoking inflammation of blood vessels cannot remain without consequences. Indeed, continued elastin receptor activation by C-peptide and GxxP fragments leaves a state of vascular overrepair, hitherto called inflammation, and otherwise called atherosclerosis, a condition characterized by thickening of blood vessel walls with activated inflammatory and blood vessel cells that underlies all conditions of metabolic syndrome. Over years, and in trickling fashion that varies from person to person, more-and-more damage is done to the elasticity and strength of our vasculature of various organs (such as heart, blood vessels, pancreas, kidney, brain) that generally leads to atherosclerosis, hypertension and dyslipidaemia, and ultimately leads to various manifestations as cardiovascular disease, diabetes type 2, chronic kidney failure and vascular dementias.

The finding explains how every day excess food intake results in overrepair of blood vessels, also called chronic inflammation. In short: every time we consume food with glucose (sugar) we produce insulin and thus C-peptide, every time we eat too much glucose, we produce more-and-more insulin, and thus more-and-more (excess) C-peptide. It is this excess C-peptide and the fragments thereof that still carry the key unlocking motif GxxP that cause excess elastin receptor activation, leading to vascular overrepair with inflammation. Overeating every day directly causes excess production of C-peptide and its GxxP-fragments that every day adds up to elastin receptor induced overrepair, leading up to the chronic overrepair and so-called inflammation, the dislipidaemia, hypertension and ultimately unhealthy blood vessels seen in metabolic syndrome. With this insight, the finding discloses roads to develop and use products (immunolatory peptides) to block GxxP lock-and-key interaction to prevent disease among which hypertension and atherosclerosis. Also, the invention explains the added risks of a sedentary live. In short: not using our unhealthy intake of sugary food as fuel for our muscles urges our bodies to produce more-and-more insulin to help the liver to change the excess sugar into fat that can comes back in the blood leaving us with dislipidaemia. Again, excess C-peptide is produced along with insulin, again setting the lock-and-key elastin receptor activation in motion, leading up to the deregulation of fat metabolism as described. Thirdly, the invention explains the added risks of smoking as well. In short: smoking (or similarly: air pollution) causes damage to the elastic tissue of the lung, thereby releasing fragments of elastin having the GxxP motif (herein called elastin peptides) which are known to cause elastin receptor activation. Thus, smoking adds more peptides with the GxxP motif to the already circulating C- peptide fragments with that motif. This accumulation of diet-induced C-peptide and smoking induced elastin peptide ads up to aggravated overrepair and so-called inflammation and thus aggravated cardiovascular or chronic kidney disease in those people that both smoke and indulge in too much sugar from their diet. The invention is showing an as yet fully unknown common causal relationship between diseases caused by different lifestyle conditions, overeating, being sedentary and smoking. The invention provides peptides to block GxxP lock-and key interaction to prevent disease. Both C-peptide and elastin peptides, and their breakdown products or fragments are relatively stable in blood and urine. Where insulin is rapidly degraded in the liver and disappears from the blood, C-peptide (and breakdown fragments with the GxxP motif) as well as elastin peptides have a much longer life in the blood and are only excreted by the kidney. Thus, whether eating-on and producing new insulin with new C-peptide, or continuing smoking and producing more elastin decay of the lungs; levels of C-peptide and elastin peptides build up and over time cause more and more vascular damage via GxxP-mediated activation of the elastin receptor. GxxP lock-and key interaction of both C-peptide and elastin peptides may be blocked with appropriate peptides to prevent disease depending on the outcome of these diagnostic tests as disclosed herein. Also, the invention provides peptides that stimulate (agonists) or inhibit (antagonists) GxxP-receptor binding. Agonists may be used in patients wherein C-peptide levels are low, such as in people suffering from diabetes type 1 or end-phase diabetes type 2. Antagonists may be used in patients wherein C-peptide levels are high, in particular in people suffering or prone to be suffering from metabolic syndrome.

. In a further embodiment, said product is provided with at least one additional active agent effective to treat microvascular disease. Such a product as provided herein may be used for treating nephropathy or for preparing a medicament for treating nephropathy, or for treating neuropathy or for preparing a medicament for treating neuropathy, or for treating retinopathy or for preparing a medicament for treating retinopathy. Such a product may additionally be used or provided with an agent for glycemic control, preferably insulin or an antidiabetic agent functionally equivalent to insulin, preferably wherein the antidiabetic agent comprises regular insulin or an insulin analogue such as insulin lispro, insulin glulisine, insulin aspart, insulin degludec, insulin glargine, or wherein the antidiabetic agent comprises a sulfonylurea or a meglitinide or metformin.

Synthetic peptides with amino acid sequence motif GxxP, allowing a type VIII beta-turn, preferably wherein said site comprises an amino acid sequence motif GxxPG, as provided herein are useful in the treatment of inflammatory conditions, such as acute kidney injury, also in acute systemic inflammatory conditions such as for example sepsis or systemic inflammatory response syndrome (SIRS), leading to vascular damage and often aggravated by (multiple organ) organ failure, or inflammatory conditions with diabetes, when given with an anti-diabetic composition such as insulin. In a further embodiment of the invention, peptides with amino acid sequence motif GxxP, allowing a type VIII beta-turn, preferably wherein said site comprises an amino acid sequence motif GxxPG, as provided herein are encapsulated in an acid resistant capsule. Such (pharmaceutical) capsules are widely used in the pharmaceutical field as oral dosage forms for administration to humans and animals. Filled with a peptide according to the invention, such a capsule is useful for the enteral administration of a synthetic peptide provided with at least one, preferably two or three pentapeptide motifs GxxPG or xGxPG (G being glycine, P being proline, and x any amino acid), preferably wherein at least one amino acid at one position x is selected from the group of glycine, alanine, leucine, valine or isoleucine, said peptide also provided with at least one glutamine. Such administration would alleviate or treat diseases such as Crohn's disease in which gut endothelial cells need regeneration. Also, such administration would be useful in treating gastro-intestinal damage obtained after excess radiation. The peptides with amino acid sequence motif GxxP, allowing a type VIII beta-turn, preferably wherein said site comprises an amino acid sequence motif GxxPG, as provided herein may also be advantageously combined with other therapeutic immunomodulators, such as with immunomodulatory peptides, such as peptides with SEQ ID NO: 1 (LQGV), AQG, or SEQ ID NO: 2 (AQGV), or with other immunomodulators, such as with immunomodulatory antibodies or proteins directed against cytokines as TNF-alpha, IL-1 or IL-6.

### Figure legends

### Figure 1

Ido et al. (Science, 1997 Jul 25;277(5325):563-6) show human C-peptide's midportion SEQ ID NO: 8 (GGGPGAG) to normalize glucose-induced vascular dysfunction. However, finding reverse (retro) and all-D-amino acid (enantio or inverso) C-peptides equipotent to native C-peptide, they conclude the activity of SEQ ID NO: 8 (GGGPGAG) to be not mediated by a receptor, thereby teaching away from a receptor for C-peptide. This specification shows that the opposite is a case. All peptides with the midportion motif GxxP (SEQ ID NO: 38 (GGGP) in human and rat C-peptide, SEQ ID NO: 39 (GAGP) in reverse C-peptide and stereochemically equivalent PGAG in D-form C-peptide) normalize vascular dysfunction while none of the peptides without that motif do. Thus, the EBP-binding motif GxxP in SEQ ID NO: 8 (GGGPGAG) is both necessary and sufficient to normalize vascular dysfunction. Hence, human C-peptide can be considered a ligand of the EBP that modulates vascular repair via the ERC. Efficacy is expressed as an average percent of the effect of 100 nM human C-peptide. Significantly different for 30 mM glucose: ^{∗}P < 0.05. GxxP motifs in peptides bind to the elastin receptor when allowing for a close to a type VIII beta-turn confirmation, a condition considered always to be met by the motif xGxxPG. All peptides having the GxxP motif (SEQ ID NO: 38 (GGGP) or SEQ ID NO: 39 (GAGP), or all-D PGAG which is stereometrically equivalent to all-L-SEQ ID NO: 39 (GAGP)) show significant normalization of vascular dysfunction while none of the peptides without the motif show significant effects, illustrating that the elastin receptor binding motif GxxP is both necessary and sufficient to elicit the biological activity of C-peptide. Figure adapted from Ido Y, et al.

Prevention of vascular and neural dysfunction in diabetic rats by C-peptide. Science 1997; 277: 563-66.

### Figure 2

Summarized description of pathological vascular effects of GxxP-peptide deficiency versus GxxP-excess

### Figure 3

Various GxxP hexa-peptides were docked in the peptide-binding site of the elastin binding protein (EBP) using Vina/Autodock and PyMOL (1, 2, 3). The binding conformation of each peptide was chosen from the top 20 best scoring poses. A homology model of EBP (4) was used as receptor in the docking procedure. Peptides tested were:
SEQ ID NO: 41 (VGVAPG) (prototype GxxP-peptide ligand of EBP (4))
SEQ ID NO: 34 (LGGGPG) (selected from human C-peptide (5))
SEQ ID NO: 43 (QGQLPG) (immunomodulatory peptide provided herein)
SEQ ID NO: 44 (PGAYPG) (selected from human Galectin-3 (6))
SEQ ID NO: 45 (QGVLPA) (selected from loop 2 of human beta-hCG (7))

References: 1 Trott, O & Olson, AJ (2010) AutoDock Vina: improving the speed and accuracy of docking with a new scoring function, efficient optimization and multithreading, J Comp Chem 31: 455-461.
2 Seeliger, D & de Groot, BL (2010) Ligand docking and binding site analysis with PyMOL and Autodock/Vina. J Comput-Aided Mol Des 24 :417-422.
3 www.pymol.org
4 Blanchevoye, C et al. (2013) Interaction between the elastin peptide VGVAPG and human elastin binding protein, J Biol Chem 288:1317-28.
5 Ido, Y et al. (1997) Prevention of vascular and neural dysfunction in diabetic rats by C-peptide Science 277:563-6
6 De Boer, R et al. (2011) Plasma Galectin-3 Is Associated with Near-Term Rehospitalization in Heart Failure: A Meta-Analysis Journal of Cardiac Failure Vol 17, Issue 8, S93
7 Khan, NA et al. (2010) Mitigation of septic shock in mice and rhesus monkeys by human chorionic gonadotrophin-related peptides, Clin Exp Immunol 160:465-478

Similarly, All-D-amino acid peptide GPGAG fits in the model of EBP designed for docking prototype elastin peptide SEQ ID NO: 41 (VGVAPG) as well. Also, L-amino acid peptides SEQ ID NO: 40 (GGGPG) and SEQ ID NO: 46 (GAGPG) fit the model as well. EBP-associated bioactivity is considered to depend on whether the GXXP-peptide can adapt to a type VIII beta-turn confirmation at the proline (P).

### Figure 4

Overview of pathophysiological pathways in metabolic syndrome, atherosclerosis and diabetes

### Figure 5

This new perspective sheds new light on diseases that are associated with atherosclerosis and insulin resistence (e.g. cardiovascular disease, stroke, peripheral arterial disease, dementia, chronic kidney disease and beta cell failure leading to diabetes). It not only ties together sugar and smoking but also various other co-existing risk factors that result from diet (excess intake of refined starches or of processed meat products with excess elastin), or lifestyle (exposure to smog or lack of exercise). The invention puts elastin receptor activation by C-peptide forward as cause of insulin resistance, hypertension and chronic-low inflammation or blood vessel over repair in metabolic syndrome and ties this syndrome together with other conditions of insulin resistance, such as COPD due to smoking and exposure to fine particular matter, where elastin-derived peptides may activate the elastin receptor to cause insulin resistance over-repair.

### Further description of disclosures

The application describes the presence of a canonical elastin receptor binding motif, GxxP or xGxxPG, in human C-peptide, a fact that has been overlooked by the medical community at large. What is more, the motif is located in a hydrophobic midportion of C-peptide which was already in 1997 identified as central to its biological activity, but rejected as possible receptor binding site. This application shows that rejection to be invalid. The invention puts elastin receptor activation by C-peptide forward as cause of insulin resistance, hypertension and chronic-low over-repair in metabolic syndrome and ties this syndrome together with other conditions of insulin resistance, such as COPD due to smoking and exposure to fine particular matter, where elastin-derived peptides may activate the elastin receptor to cause insulin resistance and over-repair.

All amino acid sequences herein are depicted in the one-letter-code. C-peptide is found with all mammals that produce insulin, as it is co-produced and co-excreted with insulin by -cells of the pancreas. Common human C-peptide's amino acid sequence is SEQ ID NO: 1 (EAEDLQVGQVELGGGPGAGSLQPLALEGSLQ). It is herein disclosed that C-peptides from a wide variety of species bear elastin receptor binding motifs within their hydrophobic midportion (in humans SEQ ID NO: 8 (GGGPGAG)).

A receptor for C-peptide is herein identified as the elastin binding protein, which can be found in the elastin receptor complex as the alternatively spliced galactosidase derived from beta-galactosidase, encoded by the GLB1 gene (Ubiprot identifier P16278). The isoform 1 of the gene product relates to the beta galactosidase (beta-Gal) whereas the isoform 2 relates to the alternatively spliced galactosidase (S-Gal). http://www.piphuman.eu/site/home.html Beta-Gal (isoform 1) cleaves beta-linked terminal galactosyl residues from gangliosides, glycoproteins, and glycosaminoglycans, and is located mainly in the lysosomes.

Isoform 2 (S-Gal) has little or no beta-galactosidase catalytic activity, but plays functional roles in the formation of extracellular elastic fibers (elastogenesis) and in the development of connective tissue. S-Gal is considered identical to the elastin-binding protein (EBP), a major component of the non-integrin cell surface receptor expressed on fibroblasts, smooth muscle cells, chondroblasts, leukocytes, and certain cancer cell types. In elastin producing cells, EBP associates with tropoelastin intracellularly and functions as a recycling molecular chaperone that facilitates the secretions of tropoelastin and its assembly into elastic fibers.

By way of non-limiting theory as to function, damage to and destruction of the beta-cells is not only causal in type-1 diabetes but is also seen in the development of diabetes types 1.5 and 2, and metabolic syndrome as a whole as well, and the phenomena seen with insulin resistance are secondary or parallel to initial events in the pancreatic beta-cells. The damage to and destruction of the beta-cells is primarily caused by an overproduction of C-peptide that is secreted by these cells, deposited in its periphery, and leading to low-grade and initially heterogenic chronic inflammation of beta-cells and islets of Langerhans by C-peptides interaction with cells bearing the elastin receptor, an interaction mediated in human C-peptides by binding of the receptor to the hydrophobic midportion SEQ ID NO: 32 (LGGGPGAG). Before, during or after these initial events or in conjunction therewith and when overproduction of C-peptide is maintained, low-grade inflammation is extended to peripheral tissues where C-peptide is deposited as well and again cells bearing the elastin receptor are stimulated.

Further disclosed are peptides derived from the human C-peptide (Ulniprot identifier >sp|P01308|57-87) or functional mammalian equivalents thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif. Note that no stereoisomers of glycine exist, herein L-glycine and D-glycine both stand for glycine. By way of non-limiting example, the peptides may be synthesized by a solid-phase method with an automated peptide synthesizer (such as model 990; Beckman Instrument, Fullerton, CA). The peptides may be purified by reverse phase high-performance liquid chromatography (such as Capcell Pak C-18, Shiseido, Tokyo, Japan). The sequence of the peptide may be confirmed with a mass spectrometer (such as Voyager, Linear-DE/K, Preseptive Biosystems, TX).

An exemplary octapeptide as disclosed herein comprising the xGxP or GxxP motif derived from the human C-peptide is the octapeptide SEQ ID NO: 32 (LGGGPGAG) that is selected from the human C-peptide sequence as a whole and very well suited for human use considering its 100% homology over the stretch of 8 amino acids in C-peptide from which it is derived. Also provided is the retro-inverso variant, the all-D-amino acid peptide GAGPGGGL, and peptides or peptidometics of at most 30, preferably of at most 25, preferably of at most 20, preferably of at most 12, preferably of at most 9 amino acids, comprising the all-D-amino acid peptide GAGPGGGL. Note: no stereoisomers of glycine exist, here (and in retro-inverso peptides bearing for example retro-inverso GxxP or xGxP motifs) G is not, whereas other amino acids, such as L, P and A are, instrumental to the all-D-amino acid character of the retro-inverso peptide herein provided.

An exemplary hexapeptide as disclosed herein comprising the xGxP or GxxP motif derived from the human C-peptide is the hexapeptide SEQ ID NO: 8 (GGGPGAG) that is selected from the human C-peptide sequence as a whole and very well suited for human use considering its 100% homology over the stretch of 7 amino acids in C-peptide from which it is derived. Also provided is the retro-inverso variant, the all-D-amino acid peptide GAGPGGG, and peptides or peptidometics of at most 30, preferably of at most 25, preferably of at most 20, preferably of at most 12, preferably of at most 8 amino acids, comprising the all-D-amino acid peptide GAGPGGG.

Another exemplary hexapeptide as disclosed herein comprising the xGxP or GxxP motif derived from the human C-peptide is the heptapeptide SEQ ID NO: 47 (LGGGPGA) that is selected from the human C-peptide sequence as a whole and very well suited for human use considering its 100% homology over the stretch of 7 amino acids in C-peptide from which it is derived. Also provided is the retro-inverso variant, the all-D-amino acid peptide GAGPGGG, and peptides or peptidometics of at most 30, preferably of at most 25, preferably of at most 20, preferably of at most 12, preferably of at most 9, preferably of at most 7 amino acids, comprising the all-D-amino acid peptide AGPGGGL.

A most exemplary heptapeptide as disclosed herein comprising the xGxP or GxxP motif derived from the human C-peptide is the hexapeptide SEQ ID NO: 48 (GGPGAG) that is selected from the human C-peptide sequence as a whole and very well suited for human use considering its 100% homology over the stretch of 6 amino acids in C-peptide from which it is derived. Also provided is the retro-inverso variant, the all-D-amino acid peptide GAGPGG, and peptides or peptidometics of at most 30, preferably of at most 25, preferably of at most 20, preferably of at most 12, preferably of at most 9, preferably of at most 6 amino acids, comprising the all-D-amino acid peptide GAGPGG.

Another exemplary heptapeptide as disclosed herein comprising the xGxP or GxxP motif derived from the human C-peptide is the heptapeptide SEQ ID NO: 34 (LGGGPG) that is selected from the human C-peptide sequence as a whole and very well suited for human use considering its 100% homology over the stretch of 6 amino acids in C-peptide from which it is derived. Also provided is the retro-inverso variant, the all-D-amino acid peptide GPGGGL, and peptides or peptidometics of at most 30, preferably of at most 25, preferably of at most 20, preferably of at most 12, preferably of at most 9, preferably of at most 6 amino acids, comprising the all-D-amino acid peptide GPGGGL.

Another exemplary heptapeptide as disclosed herein comprising the xGxP or GxxP motif derived from the human C-peptide is the hexapeptide SEQ ID NO: 49 (GGGPGA) that is selected from the human C-peptide sequence as a whole and very well suited for human use considering its 100% homology over the stretch of 6 amino acids in C-peptide from which it is derived. Also provided is the retro-inverso variant, the all-D-amino acid peptide AGPGGG, and peptides or peptidometics of at most 30, preferably of at most 25, preferably of at most 20, preferably of at most 12, preferably of at most 9, preferably of at most 6 amino acids, comprising the all-D-amino acid peptide AGPGGG.

A most exemplary pentapeptide as disclosed herein comprising the xGxP or GxxP motif derived from the human C-peptide is the heptapeptide SEQ ID NO: 40 (GGGPG) that is selected from the human C-peptide sequence as a whole and very well suited for human use considering its 100% homology over the stretch of 6 amino acids in C-peptide from which it is derived. Also provided is the retro-inverso variant, the all-D-amino acid peptide GPGGG, and peptides or peptidometics of at most 30, preferably of at most 25, preferably of at most 20, preferably of at most 12, preferably of at most 9, preferably of at most 5 amino acids, comprising the all-D-amino acid peptide GPGGG.

Another exemplary pentapeptide is peptide SEQ ID NO: 46 (GAGPG), and peptides or peptidometics of at most 30, preferably of at most 25, preferably of at most 20, preferably of at most 12, preferably of at most 9, preferably of at most 5 amino acids, comprising the peptide SEQ ID NO: 46 (GAGPG).

Another exemplary pentapeptide is a retro-inverso variant, the all-D-amino acid peptide GPGAG, and peptides or peptidometics of at most 30, preferably of at most 25, preferably of at most 20, preferably of at most 12, preferably of at most 9, preferably of at most 5 amino acids, comprising the all-D-amino acid peptide GPGAG.

A most exemplary tetrapeptide as disclosed herein comprising the xGxP or GxxP motif derived from the human C-peptide is the tetrapeptide SEQ ID NO: 38 (GGGP) that is selected from the human C-peptide sequence as a whole and very well suited for human use considering its 100% homology over the stretch of 6 amino acids in C-peptide from which it is derived. Also provided is the retro-inverso variant, the all-D-amino acid peptide PGGG, and peptides or peptidometics of at most 30, preferably of at most 25, preferably of at most 20, preferably of at most 12, preferably of at most 9, preferably of at most 4 amino acids, comprising the all-D-amino acid peptide PGGG.

Another tetrapeptide asdisclosed herein comprising the GxxP motif is the tetrapeptide SEQ ID NO: 39 (GAGP). Also provided is the retro-inverso variant, the all-D-amino acid peptide PGAG, and peptides or peptidometics of at most 30, preferably of at most 25, preferably of at most 20, preferably of at most 12, preferably of at most 9, preferably of at most 4 amino acids, comprising the all-D-amino acid peptide PGAG.

In beta-cells, insulin is produced in conjunction with C-peptide from the same precursor molecule, preproinsuline. Both insulin and C-peptide are excreted in equal amounts into the blood, whereby insulin can act on peripheral tissues for a short time only, having a half-life of approximately 5 minutes. C-peptide, however, has a reported half-life of 30 minutes, and circulates much longer in the blood. Although C-peptide's function has long not been understood, many activities are currently been ascribed to it among which are pro-inflammatory activities. The source of theses pro-inflammatory activities has not been explained. As provided herein, structural analysis of C-peptide reveals the presence of an elastin receptor-binding motif, which, without being bound to a particular theory, is the cause of the pro-inflammatory effect.

Common human C-peptide's amino acid sequence is: SEQ ID NO: 1 (EAEDLQVGQVELGGGPGAGSLQPLALEGSLQ). The mid-portion, SEQ ID NO: 42 (ELGGGPGAGS), bears an additional, hitherto unnoticed, characteristic PG-domain that explains the so-called pro-inflammatory character of C-peptide, and in particular explains the low-grade, and initially heterogenic, chronic inflammation (herein identified as blood vessel over-repair) that is seen with diabetes type-2 and metabolic syndrome, and more in particular, explains the onset, the etiology, of diabetes type-2 as a whole. It is herein recognized that SEQ ID NO: 42 (ELGGGPGAGS) bears a canonical xGxP, GxxP, GxxPG and xGxPG (x being any amino acid, preferably a hydrophobic amino acid) sequence found in peptides reactive with the elastin binding protein, EBP. The elastin binding protein (EBP), a spliced variant of lysosomal beta-galactosidase, is the primary receptor of elastin peptides that for example have been linked to emphysema, aneurism and cancer progression. The sequences recognized by EBP share the GxxP consensus pattern found in numerous matrix proteins, notably in elastin where the SEQ ID NO: 41 (VGVAPG) motif is repeated. Herein, C-peptide is recognized for the first time as being a ligand of EBP or the elastin receptor. C-peptide thus has a same set of chemotaxic, matrix-metallo-proteinase (MMP) activating, proliferative and low-inflammatory or vascular repair activities that known elastin-peptides derived from extra-cellular matrix (ECM) proteins have. This receptor interaction has not publicly been recognized before, neither by persons skilled in the art of diabetes research or of metabolic disorder research, nor by those skilled in the art of ECM or elastin peptide research.

Having identified a binding site EBP in C-peptide, the vascular repair or low-inflammatory modulation by C-peptide is now explained. Pericytes, smooth muscle cells, fibroblasts, adipose tissue cells, pancreatic stellate cells, and other connective tissue cells, together with endothelial cells, and circulating innate immune cells such as leucocytes and monocytes, may respond to binding of EBP to GxxPG bearing proteins and peptides by interleukin-1-beta mediated proliferation and low-grade inflammatory activation. Analysis of the human proteome shows that proteins with multiple GxxPG motifs are highly related to the extracellular matrix (ECM). Matrix proteins with multiple GxxP, xGxP or GxxPG sites include fibrillin-1, -2, and -3, elastin, fibronectin, laminin, and several tenascins and collagens.

Secondly, circulating leucocytes and monocytes show strong chemotaxis to GxxP, xGxP, GxxPG or xGxPG bearing proteins and peptides, C-peptide will thus attract those cells to wherever C-peptide is present.

Thirdly, binding of EBP to GxxP, xGxP, xGxPG or GxxPG bearing proteins and peptides has been associated with shedding of EBP from cellular surfaces and increased presentation of the interleukin-I receptor having affinity for interleukin-1-beta, allowing for hampered endocytosis or for a continued interleukin-1-beta mediated proliferation and inflammatory activation wherever C-peptide deposits are present.

Fourthly, binding of EBP to GxxP or GxxPG bearing proteins and peptides has been associated with the activation of neuraminidase and the release of sialic acid from proteins that induces insulin resistance, in particular of adipocytes and hepatocytes.

Fifthly, binding EBP to GxxP or GxxPG bearing proteins and peptides has been associated with shedding of EBP from cellular surfaces and decreased presentation of PPCA having proteolytic activity towards endothelin-1, whereby increased endothelin-1 levels due to decreased proteolytic acitivity of PPCA result in increased hypertension.

The disclosures relate also in part to the identification of a receptor type which binds C-peptides or C-peptide fragments, thus inducing C-peptide related bioactivity associated with various disorders, such as immune disorders such as metabolic syndrome and diabetes. Such a receptor type which binds or interacts with C-peptides or C-peptide fragments as disclosed herein, is a mammalian elastin- receptor known to bind elastin peptides including but not limited to the elastin receptor complex, including a 67-kDa elastin-binding protein (EBP) identified as an spliced variant of beta-galactosidase, and related homologues and isoforms thereof, that is ubiquitously found on innate immune cells, extra cellular matrix cells, fibroblasts, vascular smooth muscle cells and certain tumor cells. Also binding these motifs are pancreatic elastases, herein understood to also have elastin binding protein type binding activity. Elastin binding protein type, typically binds to canonical xGxP, GxxP, GxxPG and xGxPG (x being any amino acid, preferably an hydrophobic amino acid) motifs in extracellular matrix proteins, such as elastin, laminins, collagen type IV, and fibrillin-1, and such a motif are herein for the first time identified in C-peptides. Elastin binding protein/elastin peptide interaction can also be found with integrins and galectin, EBP, integrins and galectins and other receptors capable of binding to xGxP, GxxP, GxxPG and xGxPG motifs herein commonly called elastin binding protein type. The identification of an "elastin receptor" which interacts with C-peptide to promote a biological response modulating associated with metabolic and immune disorders in turn provides a valuable and essential component when practicing additional embodiments, including but not necessarily limited to methods, uses and identified peptides for treating various disorders.

Other useful agonist or antagonist peptides may for example be found *in silico* employing the homology model of the elastin-binding site of human EBP. Blanchevoy et al recently build a homology model of this protein and showed docking of SEQ ID NO: 41 (VGVAPG) in this model (Blanchevoye et al, INTERACTION BETWEEN THE ELASTIN PEPTIDE VGVAPG AND HUMAN ELASTIN BINDING PROTEIN, doi: 10.1074/jbc.M112.419929 jbc.M112.419929*.;* the contents of which , such as the relevant atomic coordinates of the binding site, are herein included by reference).

### DETAILED DESCRIPTION

Human C-peptide is found a ligand of the human elastin receptor.
- Elastin receptor: shall mean a chemical group or molecule on the cell surface or in the cell interior that has an affinity for a peptide having an amino acid motif GxxP, wherein G represents the one-letter code for the amino acid glycine, P for the amino acid proline and x for any amino acid, the amino acid following P preferably allowing for a type VIII-beta turn, a condition which is met when P is C-terminally followed by a G, said elastin receptor typically represented in humans by the elastin binding protein known in the publicly accessible database Uniprot as GLB1- isoform 2 under identifier: P16278-2.
- C-peptide: shall mean a peptide typically produced by beta-cells in the pancreas together with insulin, said C-peptide represented in humans by the peptide known in the publicly accessible database Uniprot as INS - isoform 1 under identifier: P01308-1, position 57-87.

Human C-peptide, connecting immature insulin chains A and B and secreted in a 1:1 ratio with mature insulin into the portal circulation, has traditionally been considered inert, despite ever increasing evidence of its biological activity. I show that in dietary excess, excess serum C-peptide leads to chronic-low grade inflammation, insulin resistance and hypertension and is causal to metabolic syndrome. I show C-peptide carrying a hitherto unrecognized xGxxPG motif specific for binding of elastin peptides to the elastin receptor, said receptor fulfilling various roles in tissue inflammation and tissue repair. Recent findings show this receptor to promote insulin resistance, dislipidaemia, hypertension and atherogenesis, all characteristic of metabolic syndrome. This finding takes C-peptide into the limelight, tying in metabolic syndrome with other conditions of insulin resistance, such as COPD, when circulating elastin-derived peptides may combine with C-peptide to stimulate elastin receptor-mediated insulin resistance and inflammation.

### Insulin resistance

Insulin resistance (IR) is central to metabolic syndrome ^{1,2}. It occupies a crucial place in the aetiology of chronic inflammatory, lifestyle-, diet- or age-related, conditions as atherosclerotic cardiovascular disease and diabetes type 2. Hallmarks of metabolic syndrome are IR, hypertension, dyslipidaemia, hyperinsulinaemia, and impaired glucose tolerance. Uncertainties exist to the cause of IR. Simplified, the main view ^{1,3} holds chronic-low grade inflammation to drive IR and subsequent hyperinsulinaemia; a seemingly opposed view ² holds increased hyperinsulinaemia to drive IR and subsequent inflammation.

In humans in dietary excess, excess serum C-peptide causes chronic-low grade inflammation as well as IR and hypertension leading to metabolic syndrome, C-peptide being hitherto unrecognized as a ligand for the elastin receptor.

### The elastin receptor

The human elastin receptor ⁴⁻⁶ is involved in chemotaxis of leukocytes and activation of matrix-metallo-proteinases, in endothelial cell migration and angiogenesis and in proliferation of fibroblasts and vascular smooth-muscle cells. The receptor is activated by (proteolytic) fragments of extracellular matrix in granulating tissue after tissue injury or inflammation, fulfilling handyman jobs towards tissue repair.

The receptor consists of an alternatively spliced variant of human beta-galactosidase. It binds to a hexapeptide x-Gly-x-x-Pro-Gly (xGxxPG) motif in (proteolytic fragments of) extracellular matrix proteins such as elastin and fibrillin-1 ⁴. The best-known representative of the motif is hexapeptide SEQ ID NO: 41 (VGVAPG) found in (tropo)elastin, but many other biologically active peptides conforming to the signature sequence xGxxPG, generally called elastin peptides, have been reported as agonist ^{4,5}. A minimally essential sequence for biological activity is GxxP, with the peptide at P adopting a type VIII beta-turn ⁵. V14 peptide (SEQ ID NO: 131 (VVGSPSAQDEASPL)) corresponding to the binding site of the receptor, is used to antagonise elastin peptide binding ⁶.

The elastin receptor forms a complex with neuraminidase (Neu-1) and protective protein-cathepsin A (PPCA) on the cell surface ⁴. After binding to its ligand, the complex internalises to endosomal compartments in the cell and triggers numerous cellular responses. In mice, elastin peptides potentiate atherosclerosis through Neu-1 ⁷ and regulate IR ⁸ due to an interaction between Neu-1 and the insulin receptor. Moreover, in mice, PPCA is required for assembly of elastic fibres and inactivation of endothelin-1, impaired activation of endothelin-1 resulting in hypertension ⁹.

### C-peptide

Herein recognized, human C-peptide ( ₁SEQ ID NO: 1 (EAEDLQVGQVELGGGPGAGSLQPLALEGSLQ)₃₁) contains the xGxxPG motif (underlined), surprisingly identifying it as a ligand for the elastin receptor. The far reaching implications of that find are discussed below. Classically, C-peptide connects the A- and B-chain of insulin in the preproinsulin produced in pancreatic beta-cells from the insulin gene and facilitates folding and binding of chains A en B. After processing, mature insulin and C-peptide are secreted into the portal circulation. Be it under dietary frugality or excess, insulin and C-peptide are produced and secreted in equimolar concentrations. However, C-peptide's plasma half-life of ~30 min versus insulin's half-life of ~4 min ¹⁰ causes dietary excess to maintain persistently higher levels of circulating C-peptide than of insulin. The traditional view holds circulating C-peptide essentially inert and, because of its longer half-life, particularly useful as a surrogate marker of insulin release. However, accumulating evidence points at biological functions for C-peptide ¹¹⁻¹⁴. Excess C-peptide in mice experimentally elicits inflammatory effects in vasculature and around glomeruli and C-peptide is found deposited in atherosclerotic lesions of patients ¹⁵. Fasting serum C-peptide levels significantly relate to hazards of cardiovascular and overall death in non-diabetic adults ¹⁶. These recent findings establish pathophysiological importance to C-peptide in its own right.

### Pentapeptide₂₇ SEQ ID NO: 6 (EGSLQ) ₃₁

A first concerns the pentapeptide ₂₇ SEQ ID NO: 6 (EGSLQ) ₃₁, corresponding to the C-terminal five residues of human C-peptide, which mimics several effects of the full-length peptide. The pentapeptide displaces cell membrane-bound C-peptide, increases intracellular Ca(2+) and stimulates MAP kinase signalling pathways and Na(+),K(+)-ATPase ⁸. Of note, the glutamate at position 27 was shown essential to activation of alpha-enolase by C-peptide ¹⁴, hinting that the C-terminal pentapeptide site may be involved in interaction of C-peptide with alpha-enolase.

### Midportion ₁₃ SEQ ID NO: 8 (GGGPGAG) ₁₉

A second site, and main focus of this application, the midportion of human C-peptide 13 SEQ ID NO: 8 (GGGPGAG) ₁₉, was detected when structural features of C-peptide critical for mediating its effects on vascular dysfunction were investigated in a skin chamber granulation tissue model in rats ¹⁴. ₁₃SEQ ID NO: 8 (GGGPGAG) ₁₉ was shown to be central to C-peptide's biological activity. However, as synthetic reverse sequence (retro) and all-D-amino acid (enantio) C-peptides were found equipotent to native C-peptide, it was concluded then ¹⁴ that the effects of this midportion must rely on non-chiral interactions, thereby teaching away from any possible stereospecific receptor binding to ₁₃ SEQ ID NO: 8 (GGGPGAG) ₁₉. This teaching has since then dominated the literature on C-peptide. However, I here conclude that an xGxxPG elastin receptor binding motif is overlapping with C-peptide's bioactive midportion (₁SEQ ID NO: 1 (EAEDLQVGQVELGGGPGAGSLQPLALEGSLQ)₃₁), distinctly associated with effects on vascular function in granulation tissue, identifying C-peptide as a biologically active ligand of the elastin receptor.

### C-peptide receptor

Until now, a distinct C-peptide receptor is unknown. A binding study ¹² of C-peptide to human cell membranes indicates the existence of at least two C-peptide/receptor complexes, one with high affinity and low mobility and one with low affinity and high mobility and recent studies suggest alpha-enolase ¹⁷, a cell surface receptor of plasminogen, or GPR146 ¹⁸, associated with dyslipidaemia ¹⁹, as possible receptor candidates for C-peptide. Biologically active sites in C-peptide. At least two biologically active sites have been identified in the C-peptide.

### Non-chirality is revoked

Surprisingly, studying reference 14 anew, the xGxxPG motif is also present in the biologically active retro C-peptide (₁SEQ ID NO: 136 (QLSGELALPQLSGAGPGGGLEVQGVQLDEAE) ₃₁). Also, the biologically active enantio C-peptide (D--₁EAEDLQVGQVELGGGPGAGSLQPLALEGSLQ₃₁) carries the motif, being hidden as the retro-enantio sequence D--GPGAGS; retro-enantio peptides being stereometrically nearly identical as their parent peptides, maintaining overall side-chain topology albeit for different N-terminal and C-terminal endings ²⁰. These observations revoke the teaching ¹⁴ of non-chirality and instead allow for stereospecific binding of these peptides to a receptor recognising said motif: the elastin receptor. C-peptide is a species of the genus of elastin peptides.

Moreover, additional examples of fragments of the human C-peptide are provided herein and ¹⁴, all bearing a midportion hexapeptide ₁₂SEQ ID NO: 34 (LGGGPG) ₁₇, that all prevented vascular dysfunction whereas other human C-peptide fragments, wherein the hexapeptide midportion was disrupted, were found not active. Rat C-peptide, comprising a hexapeptide (₁₂ SEQ ID NO: 134 (LGGGPE) ₁₇) GxxP motif (the P allowing a type VIII beta-turn required for biological activity ⁵) was found active as well, whereas pig C-peptide (midportion ₁₂ SEQ ID NO: 135 (LGGGLG) ₁₇ ) not containing the essential P in the elastin binding motif, was found inactive. Of note, all 11 C-peptide(fragments) with the GxxP motif prevented vascular dysfunction, whereas all 5 without the motif did not, showing that even fragments of circulating C-peptide may contribute to elastin receptor activation, as long as the GxxP motif and the type VIII beta-turn is present. Human C-peptide and its xGxxPG containing fragments may thus be considered an unexpected species of the genus of a larger class of peptides: elastin peptides capable of elastin receptor activation, whereby excess C-peptide may be meddling with elastin receptor mediated tissue repair, modulating chronic-low grade inflammation, IR and hypertension. Insulin resistance extends beyond metabolic syndrome. Based on the above, I pose that in humans in dietary excess and prone to develop metabolic syndrome excess C-peptide binds to the elastin receptor, eliciting al least three effects, chronic-low grade inflammation (rather to be seen as excess vascular repair activity), insulin resistance and hypertension. The finding ties together conditions seen with metabolic syndrome with conditions possibly caused by circulating elastin degradation products, such as COPD, caused by smoking or by exposure to fine particulate matter (smog), or by physiological conditions, such as pregnancy and growth; allowing for a cumulative pathology when both C-peptide and elastin-derived peptides are increased, which provides a substantial jump in our understanding of the causes of metabolic syndrome and other lifestyle- or age-related conditions of IR. Elastin peptide/elastin receptor binding has been demonstrated for synthetic peptides such as SEQ ID NO: 41 (VGVAPG) and inhibited by antagonist V14 peptide ⁴⁻⁶. It is provided to do these tests with synthetic human C-peptide variants or fragments, provided with or without the sequence GxxP, to study binding, including classical elastin receptor antagonist V14 peptide to study inhibition of binding. Similarly, one can do the C-peptide tests in a skin chamber granulation tissue model of vascular function ¹⁴, or test synthetic, inducibly or constitutively expressed C-peptide in established models of atherosclerosis, Neu-1 mediated IR or PPCA mediated hypertension ⁷⁻⁹. For example, in a classical Boyden chamber experiment, an 100% increase of migration of CD4+ immune cells in 1% serum medium was demonstrated in vitro by C-peptide at 10 nM which CD4-migration was then inhibited, antagonized and diminished by >50% by V14-peptide at 1.3 microM, specifically demonstrating reduction of C-peptide specific biological activity by elastin receptor antagonist V14 peptide.

### REFERENCES

1 Shoelson SE, Lee J,Goldfine AB. J Clin Invest. 2006; 116:1793-1801*.*
2 Shanik MH, Xu Y, Skrha J, Dankner R, Zick Y, Roth J. Diabetes Care. 2008; Suppl 2:S262-8
3 Bolton CE, Evans M, lonescu AA, et al.,. COPD. 2007; Jun; 4(2):121-6.
4 Adair-Kirk TL, Senior RM. Int J Biochem Cell Biol. 2008;40(5-7):1101-10.
5 Blanchevoye et al,idoi: 10.1074/jbc.M112.419929 *jbc.M112.419929*
6 Robinet A, Fahem A, Cauchard JH, et al., J Cell Sci. 2005; Jan 15;118(Pt 2):343-56.
7 Gayral S, Garnotel R, Castaing-Berthou A, et al., Cardiovasc Res. 2013; Dec 19. [Epub ahead of print]
8 Blaise S, Romier B, Kawecki C, et al., Diabetes. 2013; Nov;62(11):3807-16.
9 Seyrantepe V, Hinek A, Peng J, et al.,. Circulation. 2008; Apr 15;117(15):1973-81.
10 Faber OK, Hagen C, Binder C, eta al., J Clin Invest 1978; 62 : 197-203
11 Hills CE, Brunskill NJ. Clin Sci (Lond). 2009; Apr;116(7):565-74.
12 Rigler, R., Pramanik, A. and Jonasson, P. Proc. Natl. Acad. Sci. U.S.A.1999; 96, 13318-13323
13 Johansson J, Ekberg K, Shafqat J, et al., Biochem Biophys Res Commun. 2002; Aug 2;295(5):1035-40.
14 Y, Ido Vindigni A, Chang K, et al., Science. 1997; Jul 25;277(5325):563-6.
15 Vasic D, Walcher D. IntJ Inflam. 2012; 2012:932725.
16 Patel N, Taveira TH, Choudhary G, Whitlatch H, Wu WC. J Am Heart Assoc. 2012; Dec;1(6):e003152.
17 Ishii T, Fukano K, Shimada K, et al., J Biochem. 2012; Jul;152(1):53-62.
18 Yosten GL, Kolar GR, Redlinger U, Samson WK. J Endocrinol. 2013;218(2):B1-8
19 *International Patent Application* WO2007/042792;
20 Muller S, Benkirane N, Guichard G, Van Regenmortel MH, Brown FExpert Opin Investig Drugs; 1998 Sep;7(9):1429-38.

Again, when we eat too much, we provide the beta-cells of our pancreas with continued glucose signaling to produce insulin, to harbor the ever excess glucose derived from our food in peripheral liver, muscle and fat cells. When eating in excess, we demand ever increasing insulin production from our beta-cells, and therewith demand ever increasing production of C-peptide from our beta-cells, C-peptide and insulin being produced and excreted in equal amounts. As insulin has a typical half-life of about 3-4 minutes, conditions of excess insulin may be easily coped with. However C-peptide has a much longer half-life, typically of >30 minutes, and depositions of excess C-peptide (and of partly or whole unprocessed pro-insulin) will be formed around the rim of the beta-cells and the islets of Langerhans, and also in the vascular wall of our blood-vessels. Pericytes, smooth muscle cells, fibroblasts, adipose tissue cells, pancreatic stellate cells, and others, together with endothelial cells, and possibly circulating leucocytes, respond to binding of EBP to GxxPG bearing C-peptide, thereby causing matrix-metallo-proteinase (MMP) induced hydrolysis accompanied by interleukin-1-beta mediated proliferation and subsequent low-grade inflammatory activation, in and around beta-cells in the islets of Langerhans. IL-1-beta thus drives tissue inflammation that impacts on both beta-cells functional mass and subsequently may also drive insulin sensitivity in type-2 diabetes. Binding of EBP to C-peptide's GxxPG sequences may further facilitate shedding of EBP from cellular surfaces and increased presentation of the interleukin-I receptor, allowing for a continued interleukin-1-beta mediated proliferation and inflammatory activation wherever C-peptide deposits are present, again driving insulin sensitivity in type-2 diabetes. In a patient thus developing diabetes type-2 or metabolic syndrome damage to and destruction of the beta-cells in the pancreas is following excess C-peptide production by those cells. Phenomena commonly seen as insulin resistance are then often secondary to initial events in the pancreatic beta-cells and arise out of interaction of fibroblasts, smooth muscle cells pericytes and leucocytes with vascular or peripheral C-peptide overload.

C-peptide exerts chemotactic and bioactive effects via interaction of its GXXPG and XGXPG motif with the elastin-binding protein. We here in this patent application teach that C-peptide exerts chemotactic and bioactive influence on monocytes, pericytes, smooth muscle cells, fibroblasts, and other cells via interaction with the elastin-binding protein (EBP) (Privitera et al., J. Biol. Chem. 1998; 273:6319-6326). This receptor recognizes Gly-X-X-Pro-Gly (XGXXPG) or X-Gly-X-Pro-Gly (XGXPG) motifs found in C-peptides, wherein X can be any amino acid, and preferably a hydrophobic amino acid. The identity of this receptor protein, commonly called the elastin binding protein (EBP), has been established as an enzymatically inactive, alternatively spliced variant of beta-galactosidase. EBP forms a complex with protective protein/cathepsin A (PPCA) and lysosomal sialidase (neuraminidase-1, Neu-1). As C-peptide is released in equimolar concentrations together with insulin, but has a much longer half-life, increased insulin excretion as result of increased food-intake will result in even higher C-peptide levels. This evokes an oversupply of C-peptide and deposits of the C-peptide are observed in the periphery of beta-cells and even in the (micro)vasculature where these C-peptide deposits evoke the low-grade inflammation so typical of what is commonly called insulin-resistance. GXXPG and XGXPG motif binding to the EBP induces interleukin-Ibeta mediated proliferation of vascular and connective tissue cells.

Pericytes, smooth muscle cells, fibroblasts, adipose tissue cells, pancreatic stellate cells, and others, together with endothelial cells, and circulating leucocytes, respond to binding of EBP to GxxPG or xGxPG bearing proteins and peptides by interleukin-1-beta mediated proliferation and low-grade inflammatory activation. Analysis of the human proteome shows that proteins with multiple GxxPG or xGxPG motifs are highly related to the extracellular matrix (ECM). Matrix proteins with multiple GxxPG or xGxPG sites include fibrillin-1, -2, and -3, elastin, fibronectin, laminin, and several tenascins and collagens.

Recent studies have shown that the Neu-1 component of the EBP complex is responsible for triggering cellular activation. EBP is present on many cell types, including various types of leukocytes, mesenchymal cells, vascular smooth muscle cells, and skin fibroblasts. Whereas the hexapeptide SEQ ID NO: 41 (VGVAPG), a commonly repeated sequence in human elastin, is the most well-recognized ligand for this receptor, C-peptide, galectin-3, the amino acid sequence SEQ ID NO: 25 (FRAAPLQGMLPGLLAPLRT) in human collagen 6 A3 (COL6A3, Uniprot identifier P1211) and the beta-2 loop of human choriogonadotropin (hCG) are now herein also recognized as also capable of binding to the EBP. In addition to SEQ ID NO: 41 (VGVAPG), (all elastin-derived) peptides that follow the motif GXXPG or XGXPG (where X is a hydrophobic amino acid) display chemotaxis for monocytes in vitro (Bisaccia F, et al., Int. J. Pept. Protein Res. 1994 ;44:332-341, Castiglione Morelli MA, et al., J. Pept. Res. 1997 ;49:492-499). This is noteworthy, albeit not having been observed before, because primate C-peptide sequences do not contain the SEQ ID NO: 41 (VGVAPG) sequence; however, primate C-peptide contain significant quantities of both GXXP, GXXPG and XGXPG motifs that show similar activities. C-peptide's GxxP, GxxPG and xGxPG interactions explain IL-1-beta involvement. C-peptide's GxxP, GxxPG and xGxPG interactions have until now been overlooked by those skilled in the art of diabetes or metabolic disorder research as well as by those skilled in the art of elastin peptide and extracellular matrix (ECM) research. This earlier unobserved fact explains the macrophage-predominant, IL-1-beta mediated chronic inflammatory disease process as seen in for example adipose tissue in patients suffering from diabetes type-2, it explains the intima thickening and smooth muscle cell proliferation seen in vessels of patients suffering from atherosclerosis, the direct insulitis and peri-islet inflammation around beta cells in the pancreas as seen in the early phases of diabetes, and many other disease manifestations of metabolic syndrome wherein the patients suffer from C-peptide overproduction and C-peptide deposits, likely as a consequence of over-eating. C-peptide's GxxP, GxxPG and xGxPG interactions also explain leucocyte involvement. In addition, IL-1-beta signaling results in the production of pro-inflammatory mediators that act in a feed-forward autocrine/paracrine manner in beta-cells and local innate immune cells to amplify these effects, amplified by the fact that circulating leucocytes show strong chemotaxis to GxxPG or xGxPG bearing proteins and peptides; again C-peptide will thus attract those cells to wherever C-peptide is present, and in situations of C-peptide overload or even C-peptide deposits, this will exacerbate disease. As indicated herein, the concept that C-peptide and degradation products thereof can drive a macrophage-predominant, chronic inflammatory disease process via its GxxPG and xGxPG motif is now elucidating the etiology of diabetes of all types and is applicable to all diseases that occur in vasculature-rich organs and tissues, including coronary artery disease, peripheral vascular disease, and aortic aneurysm.

By "peptide" the inventor includes not only molecules in which amino acid residues are joined by peptide (-CO-NH-) linkages but also functionally equivalent molecules in which the peptide bond is reversed. Retro-inverse peptides are composed of D-amino acids assembled in a reverse order from that of the parent L-sequence, thus maintaining the overall topology of the native sequence. Such retro-inverso peptidomimetics may be made using methods known in the art, for example such as those described in Meziere et al. (1997) J. Immunol. 159, 3230-3237, and Carver et al. (1997) Biopolymers. 1997 Apr 15; 41(5):569-90. This approach involves making pseudopeptides containing changes involving the backbone, and not the orientation of side chains. Meziere et al. and Carver et al. (1997) show that these pseudopeptides are useful. Retro-inverse peptides are much more resistant to proteolysis. Retro-inversion is a way of protecting peptide substances against proteolysis. It entails retro-inverting those peptide bonds most susceptible to enzymatic hydrolysis by inverting the direction of the peptide bonds. The "retro-inverso peptides" are structural isomers of the reference peptides and as such preserve their biological activity while being more resistant to enzymatic hydrolysis. A peptidomimetic is a small protein-like chain designed to mimic a peptide. They typically arise from modification of an existing peptide in order to alter the molecule's properties. For example, they may arise from modifications to change the molecule's stability or biological activity (only useful when the peptide's biological activity is chiral). Chemically synthesized peptides generally have free N- and C-termini. N-terminal acetylation and C-terminal amidation reduce the overall charge of a peptide; therefore, its overall solubility might decrease. However, the stability of the peptide could also be increased because the terminal acetylation/amidation generates a closer mimic of the native protein. These modifications might increase the biological activity of a peptide and are herein also provided.

### Peptide Synthesis

Synthetic PG-domain or GxxP-type peptides such as SEQ ID NO: 41 (VGVAPG), SEQ ID NO: 138 (GVAPGV), SEQ ID NO: 139 (VAPGVG), SEQ ID NO: 140 (APGVGV), SEQ ID NO: 141 (PGVGVA), SEQ ID NO: 142 (GVGVAP), SEQ ID NO: 60 (PGAIPG), SEQ ID NO: 137 (LGTIPG), SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 81 (GGIPP), SEQ ID NO: 89 (GIPPA), SEQ ID NO: 90 (GGIPPA), or retro-inverso variants thereof are synthesized according to classical solid phase synthesis. V14 peptide, a peptide reproducing the sequence of S-Gal interacting with elastin peptides bearing the PG-domain, in particular the motif GxxP, is obtained from Neosystem (Strasbourg, France). Alternatively, V14 peptide and variants thereof are synthesised as described herein. Purity of the peptides is confirmed by high performance liquid chromatography and by fast atom bombardment mass spectrometry.

Traditionally, peptides are defined as molecules that consist of between 2 and 50 amino acids, whereas proteins are made up of 50 or more amino acids. In addition, peptides tend to be less well defined in structure than proteins, which can adopt complex conformations known as secondary, tertiary, and quaternary structures. Functional distinctions may also be made between peptides and proteins. Peptides, however, may be subdivided into peptides, which have few amino acids (e.g., 2 to 30-50), and polypeptides, which have many amino acids (>50). Proteins are formed from one or more polypeptides joined together. Hence, proteins essentially are very large peptides. In fact, most researchers, as well as this application, use the term *peptide* to refer specifically to peptides, or otherwise relatively short amino acid chains, with the term *polypeptide* being used to describe proteins, or chains of > 50 or much more amino acids.

Treatment of cultured cells with C-peptide or fragments therof.

Cells may be plated at a density of 450 per mm² in a 24-well microplate or 32 mm diameter Petri dish and cultured for 2-4 days or in cultures as described above. On day 2 in culture, cells are treated with various C-peptides (preferably selected from Table 1) or peptide fragments thereof for 1 or 2 days. In an experiment, cells were treated with a combination of C-peptide (1 micro-M) or polyclonal anti-67 kDa elastin receptor antibody (anti-S-Gal antibody) (10 ng per ml) for 2 d. At the end of the treatment, cells may be trypsinized (0.25%) and the cell number determined with a Coulter counter. For determination of thymidine incorporation, cells are labeled with 50 micro-Ci of [methyl-³H] thymidine (3.2 TBq per mmol; Amersham) for the final 18 h of the treatment. Incorporated thymidine is determined as trichloroacetic acid-precipitable counts with a liquid scintillation spectrometer (Beckman LS9800). Binding may be antagonized by adding V32-peptide or V32-peptide fragments or V14 peptide or V14-peptide fragments.

Detection of the 67 kDa elastin receptor. To select for or confirm the presence of the 67 kDa elastin receptor in cells, reverse transcription-polymerase chain reaction is performed using cellular RNA and synthetic oligoprimers corresponding to the beta-galactosidase cDNA sequences upstream and downstream spanning the region between exons 2 and 5. The reaction is run for 40 cycles with denaturation at 90°C for 1 min, annealing at 50°C for 2 min, and extension at 72°C for 5 min in a DNA Thermal Cycler (Perkin-Elmer Cetus). The polymerase chain reaction products are preferably analyzed on 1% agarose gel.

In describing protein or peptide composition, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal, left terminal to right terminal, the N-terminal being identified as a first residue. Ala: alanine residue; Asp: aspartate residue; Glu: glutamate residue; Phe: phenylalanine residue; Gly: glycine residue; His: histidine residue; Ile: isoleucine residue; Lys: lysine residue; Leu: leucine residue; Met: methionine residue; Asn: asparagine residue; Pro: proline residue; Gln: glutamine residue; Arg: arginine residue; Ser: serine residue; Thr: threonine residue; Val: valine residue; Trp: tryptophane residue; Tyr: tyrosine residue; Cys: cysteine residue. The amino acids may also be referred to by their conventional one-letter code abbreviations; A=Ala; T=Thr; V=Val; C=Cys; L=Leu; Y=Tyr; I=Ile; N=Asn; P=Pro; Q=Gln; F=Phe; D=Asp; W=Trp; E=Glu; M=Met; K=Lys; G=Gly; R=Arg; S=Ser; and H=His.

### Overview 1. Elastin degradation and elastin peptides with a GxxP motif are associated with vascular disease.

Elastin-derived peptides and Elastin Receptor Complex (ERC) mediated vascular disease
Activation of ERC by proteolytically degraded elastin peptides is associated with vascular disease.
   - Matrix ageing and vascular impacts: focus on elastin fragmentation. Duca L, et al; Cardiovasc Res. 2016 Jun 1;110(3):298-308.
   - Hellenthal FA, Buurman WA, Wodzig WK, Schurink GW. Biomarkers of AAA progression. Part 1: extracellular matrix degeneration. Nat Rev Cardiol 2009;6: 464-474.
   - Monocyte chemotactic activity in human abdominal aortic aneurysms: role of elastin degradation -peptides and the 67-kD cell surface elastin receptor. Hance KA, et al; J Vasc Surg 2002;35:254 - 261.
   - Elastin degradation is associated with progressive aortic stiffening and all-cause mortality in predialysis chronic kidney disease. Smith ER, et al; Hypertension. 2012 May;59(5):973-8

### Prototype synthetic elastin peptide SEQ ID NO: 41 (VGVAPG)

Evidence that interaction of SEQ ID NO: 41 (VGVAPG) with ERC may cause atherosclerosis and is involved in macrophage chemotaxis and angiogenesis.
- Elastin-derived peptides potentiate atherosclerosis through the immune Neu1-PI3Ky pathway. Gayral S, et al; Cardiovasc Res. 2014 Apr 1;102(1):118-27.
- Induction of macrophage chemotaxis by aortic extracts from patients with Marfan syndrome is related to elastin binding protein. Guo G, et al; PLoS One. 2011;6(5): e20138.
- Elastin-derived peptides enhance angiogenesis by promoting endothelial cell migration and tubulogenesis through upregulation of MT1-MMP. Robinet A, et al; J Cell Sci. 2005 Jan 15;118 (Pt 2):343-56.

Proteolytically degraded elastin peptides SEQ ID NO: 143 (VPGVGISPEA) and SEQ ID NO: 144 (GVAPGIGPGG)
Evidence that SEQ ID NO: 143 (VPGVGISPEA) and SEQ ID NO: 144 (GVAPGIGPGG) localize in human atherosclerotic lesions and that serum levels of SEQ ID NO: 144 (GVAPGIGPGG) associate with acute myocardial infarction. Note: None of the below authors recognize the GxxP motif in SEQ ID NO: 143 (VPGVGISPEA) and SEQ ID NO: 144 (GVAPGIGPGG)
   - Acute Myocardial Infarction and Pulmonary Diseases Result in Two Different Degradation Profiles of Elastin as Quantified by Two Novel ELISAs. Skjøt-Arkil H, et al; PLoS One. 2013 Jun 21;8(6):e60936.
Additional elastin derived peptides that interact with ERC and have biological activity are extensively discussed in:
   - Degradation of tropoelastin by matrix metalloproteinases--cleavage site specificities and release of matrikines. Heinz A, et al; FEBS J. 2010 Apr;277(8):1939-56

### Overview 2. Non-elastin peptides that have a GxxP-motif and are associated with vascular disease.

C-peptide with midportion SEQ ID NO: 8 (GGGPGAG)
Evidence that C-peptide localizes in human atherosclerotic lesions, induces macrophage chemotaxis and angiogenesis, that C-peptide may cause atherosclerosis and that serum levels of C-peptide associate with overall, cardiovascular and diabetes mortality. Typical degradation products of C-peptide are SEQ ID NO: 145 (VELGGGPGAGSLQP), SEQ ID NO: 146 (LGGGPGAGSLQP) and SEQ ID NO: 147 (LGGGPGAGS). Note: None of the below authors recognize the GxxP motif in C-peptide.
   - C-peptide colocalizes with macrophages in early arteriosclerotic lesions of diabetic subjects and induces monocyte chemotaxis in vitro. Marx N, et al; Arterioscler Thromb Vasc Biol. 2004 Mar;24(3):540-5.
   - Proinsulin C-peptide prevents impaired wound healing by activating angiogenesis in diabetes. Lim YC, et al; J Invest Dermatol. 2015 Jan;135(1):269-78.
   - C-peptide promotes lesion development in a mouse model of arteriosclerosis. Vasic D, et al; J Cell Mol Med. 2012 Apr;16(4):927-35.
   - Fasting serum C-peptide levels predict cardiovascular and overall death in nondiabetic adults. Patel N, et al; J Am Heart Assoc. 2012 Dec;1(6): e003152.
   - C-peptide levels are associated with mortality and cardiovascular mortality in patients undergoing angiography: the LURIC study. Marx N, et al; Diabetes Care. 2013 Mar;36(3):708-14.
   - Serum C-peptide levels and risk of death among adults without diabetes mellitus. Min JY, Min KB.
CMAJ. 2013 Jun 11;185(9):E402-8.
   - Serum C-peptide levels as an independent predictor of diabetes mellitus mortality in non-diabetic individuals. Min JY, Min KB. Eur J Epidemiol. 2013 Sep;28(9):771-4.
Galectin-3 with N-terminal "collagen-like-stretch" SEQ ID NO: 148 (AGAGGYPGASYPGAYPGQAPPGAYPGQAPPGAYPGAPGAYPGAPAPGVYPGPPSG)
Evidence that galectin-3 plasma levels associate with heart failure. Note: None of the below authors recognize the GxxP motif in galectin-3.
   - Galectin-3, a novel marker of macrophage activity, predicts outcome in patients with stable chronic heart failure. Van der Lok, D, et al; J Am Coll Cardiol 2007 49Suppl. A 98A[Abstract]
   - Predictive value of plasma galectin-3 levels in heart failure with reduced and preserved ejection fraction. de Boer RA, et al; Ann Med. 2011 Feb;43(1):60-8.
Fibrillinin-1 with motif SEQ ID NO: 149 (EGFEPG)
Evidence that interaction of SEQ ID NO: 149 (EGFEPG) with ERC is involved in macrophage chemotaxis.
   - Induction of macrophage chemotaxis by aortic extracts of the mgR Marfan mouse model and a GxxPG-containing fibrillin-1 fragment. Guo G, et al; Circulation 2006; 114:1855 -1862.
Laminin with motif SEQ ID NO: 137 (LGTIPG)

Evidence that laminin interacts via motif SEQ ID NO: 137 (LGTIPG) with ERC and induces fibroblast and tumor cell chemotaxis.
- The elastin receptor shows structural and functional similarities to the 67-kDa tumor cell laminin receptor. Mecham RP et al; J Biol Chem. 1989 Oct 5;264(28):16652-7.

### Table 1

**TABLE 1: C-peptide, interspecies comparisons and alignments**

| Species | Uniprot identifier | C-peptide amino acid sequence |
|---|---|---|
| Human | >sp\|P01308\|57-87 | SEQ ID NO: 1 (EAEDLQVGQVELGGGPGAGSLQPLALEGSLQ) |
| human variant | rs121908279 | SEQ ID NO: 150 (EAEDLQVGQVEMGGGPGAGSLQPLALEGSLQ) |
| human variant | rs121908274 | SEQ ID NO: 151 (EAEDLQVGQVELGGGPGAGSLQPLALERSLQ) |
| chimpanzee | >sp\|P30410\|57-87 | SEQ ID NO: 1 (EAEDLQVGQVELGGGPGAGSLQPLALEGSLQ) |
| Gorilla | >sp\|Q6YK33\|57-87 | SEQ ID NO: 1 (EAEDLQVGQVELGGGPGAGSLQPLALEGSLQ) |
| orangutan | >sp\|Q8HXV2\| 57-87 | SEQ ID NO: 1 (EAEDLQVGQVELGGGPGAGSLQPLALEGSLQ) |
| Gibbon | G1RSS5 | SEQ ID NO: 152 (EAEDPQVGQVELGGGPGAGSLQPLALEGSLQ) |
| macaque | >sp\|P30406\|57-87 | SEQ ID NO: 152 (EAEDPQVGQVELGGGPGAGSLQPLALEGSLQ) |
| green monkey | >sp\|P30407\|57-87 | SEQ ID NO: 152 (EAEDPQVGQVELGGGPGAGSLQPLALEGSLQ) |
| mouse insulin 2 | >sp\|P01326\|57-87 | SEQ ID NO: 153 (EVEDPQVAQLELGGGPGAGDLQTLALEVAQQ) |
| mouse insulin 1 | >sp\|P01325\|57-85 | SEQ ID NO: 167 (EVEDPQVEQLELGGSPGDLQTLALEVARQ) |
| rat insulin 2 | >sp\|P01323\|57-87 | SEQ ID NO: 154 EVEDPQVAQLELGGGPGAGDLQTLALEVARQ) |
| rat insulin 1 | >sp\|P01322\|57-87 | SEQ ID NO: 155 (EVEDPQVPQLELGGGPEAGDLQTLALEVARQ) |
| Horse | F6QQU 6 | SEQ ID NO: 156 (EAEDPQVGQEELGGGPGLGGLQPLALAGPQQ) |
| Horse | >sp\|P01310\|33-63 | SEQ ID NO: 157 (EAEDPQVGEVELGGGPGLGGLQPLALAGPQQ) |
| Horse | Most horses | SEQ ID NO: 158 (EAEDPQVGQVELGGGPGLGGLQPLALAGPQQ) |
| chinchilla | >sp\|P01327\|33-63 | SEQ ID NO: 159 (ELEDPQVGQADPGVVPEAGRLQPLALEMTLQ) |
| Guinea pig | >sp\|P01329\|57-87 | SEQ ID NO: 160 (ELEDPQVEQTELGMGLGAGGLQPLALEMALQ) |
| Rabbit | >sp\|P01311\|57-87 | SEQ ID NO: 161 (EVEELQVGQAELGGGPGAGGLQPSALELALQ) |
| Bovine | >sp\|P01317\|57-82 | SEQ ID NO: 164 (EVEGPQVGALELAGGPGAGGLEGPPQ) |
| Bovine | Fleckvieh variant | SEQ ID NO: 165 (EVEGPQVGALELAGGLGAGGLEGPPQ) |
| Sheep | >sp\|P01318\|57-82 | SEQ ID NO: 164 (EVEGPQVGALELAGGPGAGGLEGPPQ) |
| Pig | >sp\|P01315\|57-85 | SEQ ID NO: 166 (EAENPQAGAVELGGGLGGLQALALEGPPQ) |
| Dog | >sp\|P01321\|57-87 | SEQ ID NO: 162 (EVEDLQVRDVELAGAPGEGGLQPLALEGALQ) |
| Cat | >sp\|P06306\|57-87 | SEQ ID NO: 163 (EAEDLQGKDAELGEAPGAGGLQPSALEAPLQ) |

**Table 2**

| A, elastic fiber proteins | |
|---|---|
| Elastin, P15502, H. sapiens | ₅₀₁ SEQ ID NO: 170 (GLVPGVGVAPGVGVAPGVGVAPGVGLAPGVGVAPGVGVAPG) ₅₄₁ |
| Fibrillin-1, P35555, | ₄₁₁ SEQ ID NO: 168 (PVLPVPPGFPPGPQIPVPRP) ₄₃₀ - |
| H. sapiens | ₂₁₉₁ SEQ ID NO: 169 (TCEEGFEPGPM) ₂₂₀₁ |
| Fibrillin-2, P35556, | ₄₂₁ SEQ ID NO: 171 (LPMGGIPGSAGSRPGGTGGN) ₄₄₀ - |
| H. sapiens | ₂₂₃₇ SEQ ID NO: 172 (NCNEGFEPGPM) ₂₂₄₇ |

| B, C-peptides | |
|---|---|
| P01308, H. sapiens | ₅₇ SEQ ID NO: 1 (EAEDLQVGQVELGGGPGAGSLQPLALEGSLQ) ₈₇ |
| P30410, P. troglodytes | ₅₇ SEQ ID NO: 1 (EAEDLQVGQVELGGGPGAGSLQPLALEGSLQ) ₈₇ |
| Q6YK33, G. gorilla | ₅₇ SEQ ID NO: 1 (EAEDLQVGQVELGGGPGAGSLQPLALEGSLQ) ₈₇ |
| Q8HXV2, P. pygmaeus | ₅₇ SEQ ID NO: 1 (EAEDLQVGQVELGGGPGAGSLQPLALEGSLQ) ₈₇ |
| P01325, M. musculus;(Ins-1) | ₅₇ SEQ ID NO: 167 (EVEDPQVEQLELGGSPGDLQTLALEVARQ) ₈₅ |
| P01326, M. musculus;(Ins-2) | ₅₇ SEQ ID NO: 153 (EVEDPQVAQLELGGGPGAGDLQTLALEVAQQ) ₈₇ |
| P01322, R. norvegicus;(Ins-1) | ₅₇ SEQ ID NO: 155 (EVEDPQVPQLELGGGPEAGDLQTLALEVARQ) ₈₇ |
| P01323, R. norvegicus;(Ins-2) | ₅₇ SEQ ID NO: 154 (EVEDPQVAQLELGGGPGAGDLQTLALEVARQ) ₈₇ |
| Q62587, P. obesus | ₅₇ SEQ ID NO: 173 (GVDDPQMPQLELGGSPGAGDLRALALEVARQ) ₈₇ |
| G5C2F2, H. glaber | ₅₇ SEQ ID NO: 174 (ELENLQVGQAEPGMGLEAGGLQPLAQELALQ) ₈₇ |
| P01315, S. scrofa | ₅₇ SEQ ID NO: 166 (EAENPQAGAVELGGGLGGLQALALEGPPQ) ₈₅ |

Table 2 The presence of the elastin receptor binding motif GxxP (underlined) in vascular matrix proteins elastin and fibrillin and in C-peptides. Peptides are shown with their respective identifiers and amino acids are numbered as shown in the database Uniprot.

### Furthert identification of ERC-docking sites

The elastin-receptor-complex (ERC) is thought to cause human vascular disease by binding excess peptide ligands derived from proteolysis of extra-cellular-matrix (ECM) after aging or smoking. We now identified novel ERC-ligands, notably in well-known biomarkers of vascular disease C-peptide (induced with insulin by high blood-glucose) and NTproBNP (induced in cardiomyocyte stress). We propose A) to investigate accumulation of ERC-ligands as central etiology of human vascular disease, B) to early detect vascular disease risk by testing for ERC-ligands arising from accumulated risks diet, lifestyle and aging, that may all result in human vascular disease.

**Background.** ERC is a complex of elastin binding protein (EBP), protective protein/cathepsin A and neuraminidase-1, found on leucocytes, fibroblasts and smooth muscle cells. ERC-ligands confirm to binding motifs xGxxPG or xxGxPG (G being glycine, P proline, x any amino acid), or xGxxPx if adapted to a type VIII beta-turn. Prototype ERC-ligand SEQ ID NO: 41 (VGVAPG) and others, such as SEQ ID NO: 197 (YGYGPG), SEQ ID NO: 198 (YGARPG), SEQ ID NO: 199 (FGAVPG), are derived by proteolysis from repeat areas in elastin. Others are SEQ ID NO: 149 (EGFEPG) (fibrilin) and SEQ ID NO: 137 (LGTIPG) (laminin). EBP separately binds galactosides. ERC-ligand binding to EBP is antagonized by V14 peptide. Circulating levels of ERC-ligands, generated from elastin proteolysis in aging or by smoking, have been associated with atherosclerosis, arterial stiffness, abdominal aortic aneurysms and myocardial infarction in humans, providing ample basis to explore early diagnosis, prevention and treatment of ERC-mediated vascular disease. A composite *in silico* model is available to dock ERC-ligands in EBP for structural analyses and candidate drug-development. *In vitro,* ERC-ligand/EBP structure-function relationship may be studied in human cells by testing leukocyte chemotaxis, and proliferation of smooth muscle cells. ERC-ligands induce atherosclerosis and resistance to insulin in mice allowing *in vivo* study of ERC-mediated vascular disease.

### Identification of ERC-ligand motifs derived by proteolysis from non-ECM proteins.

- A first find is C-peptide, a peptide derived by prohormone convertase cleavage (PC) from the pre-proinsulin gene and excreted in equimolar amounts with insulin. C-peptide carries the ERC-ligand motif SEQ ID NO: 34 (LGGGPG). Ido et al how C-peptide fragments with core motif SEQ ID NO: 8 (GGGPGAG) to mitigate glucose-induced vascular dysfunction in rats but do not recognize the ERC-ligand motif. C-peptide has been found atherogenic in mice and an independent marker of human vascular disease. Thus, finding a putative ERC-ligand SEQ ID NO: 34 (LGGGPG) in C-peptide acutely links ERC-mediated vascular disease to high circulating C-peptide levels. It surprisingly provides a common etiology of vascular disease after smoking as well as after diets high in glucose or starch, wherein both etiologies are causally linked to circulating ligands of ERC.
- A second find is galectin-3, which has an N-terminal domain, susceptible to proteolysis, with putative ERC-ligand repeat motifs SEQ ID NO: 44 (PGAYPG). Galectin-3 is an independent marker of human vascular disease as well as obesity that underlies vascular disease. As galectin-3 and EBP both bind galactosides and are causal to insulin resistance in mice we suggest a second relationship of galectin-3 to EBP next to putative ERC-ligand-receptor interaction.
- A third find is ERC-ligand peptide motif SEQ ID NO: 45 (QGVLPA) in loop 2 of beta-chorionic gonadotropin (beta-hCG), expressed during pregnancy, which loop is nicked by proteolysis from beta-hCG and involved in immunomodulation and angiogenesis.
- We docked newly found SEQ ID NO: 34 (LGGGPG), SEQ ID NO: 44 (PGAYPG) and SEQ ID NO: 45 (QGVLPA), and prototype SEQ ID NO: 41 (VGVAPG), in the *in-silico* model of EBP. All fit this composite model. Also, preliminary *in-vitro* results show inhibition of bioactivity of C-peptide by ERC-antagonists V14 peptide.
- We then performed a further search for proteins with xGxxPG or xxGxPG motifs closely flanked by PC cleavage sites, to identify ERC-ligands in refulatory model elements rf fragments thereof that may derive from pro-proteins. We found SEQ ID NO: 200 (GVGAPG), SEQ ID NO: 186 (PLGSPG), SEQ ID NO: 201 (DGAKPG), SEQ ID NO: 202 (QGMLPG), and SEQ ID NO: 196 (AGGAPG) in procalcitonin (PCT), amino-terminal pro-brain natriuretic peptide (NTproBNP), pro-opiomelanacortin (POMC), collagen 6A3 (COL6A3), and pyrin, respectively. PCT and NTproBNP each correlate with heart failure. POMC relates to regulation of feeding behavior and COL6A3 relates to adipocyte function in obesity and insulin resistance. Pyrin relates to innate immunity.

**Table 3 Biomarkers of vascular disease that carry the elastin receptor binding motif**

| Table 3 Biomarkers of vascular disease that carry the elastin receptor binding motif | **name** | relevant in silico hexa- fit in EBP peptide |
|---|---|---|
| Multiple occurences of docking motif | | SEQ ID NO: 41 (VGVAPG) + |
| SEQ ID NO: 216 (VGVAPGVGVAPGVGVAPGVGLAPGVGVAPGVGVAPGVGVAPG) | Elastin | |
| SEQ ID NO: 203 (FGLVPGVGVA) | | SEQ ID NO: 214 (FGLVPG) |
| SEQ ID NO: 144 (GVAPGIGPGG) | Elastin after MMP9/12 | SEQ ID NO: 215 (PGIGPG) |
| SEQ ID NO: 205 (PPGAYPGQAPPGAYPGAPGAYPGAPAPG) | Galectin-3 | SEQ ID NO: 44 (PGAYPG) + |
| Single occurence of docking motif | | SEQ ID NO: 149 (EGFEPG) |
| SEQ ID NO: 206 (TCEEGFEPGP) | Fibrillin-1 | |
| SEQ ID NO: 207 (NPLGTIPGGN) | Laminin beta-1 | SEQ ID NO: 137 (LGTIPG) |
| Single occurence of docking motif regulatory model element peptide | | |
| SEQ ID NO: 208 (RREAEDLQVGQVELGGGPGAGSLQPLALEGSLQKR) | proinsulin C-peptide | SEQ ID NO: 34 (LGGGPG) + |
| SEQ ID NO: 209 (RVLQGVLPALPQVVCNYR) | beta-hCG loop 2 | SEQ ID NO: 45 (QGVLPA) + |
| SEQ ID NO: 210 (KRCGNLSTCMLGTYTQDFNKFHTFPQTAIGVGAPGKKR) | Procalcitonin | SEQ ID NO: 200 (GVGAPG) |
| SEQ ID NO: 211 (RSHPLGSPGSASDLETSGLQEQR) | NT-proBNP | SEQ ID NO: 186 (PLGSPG) |
| SEQ ID NO: 212 (KREDVSAGEDCGPLPEGGPEPRSDGAKPGPREGKR) | Pro-opiomelanacortin | SEQ ID NO: 201 (DGAKPG) |
| SEQ ID NO: 213 (RAAPLQGMLPGLLAPLR) | Collagen 6A3 | SEQ ID NO: 202 (QGMLPG) |
| SEQ ID NO: 192 (RRNASSAGRLQGLAGGAPGQKECR) | Pyrin | SEQ ID NO: 196 (AGGAPG) |

We found that three well-known circulating biomarkers of vascular disease, C-peptide, amino-terminal pro-B-type natriuretic peptide (NT-proBNP) and galectin-3, and others, share a little-known docking site with circulating elastin-derived-peptides (EDP). Through this docking site, EDP activate the elastin-receptor-complex (ERC) that is expressed on cells throughout the human arterial system. ERC contributes to elastin degradation and arterial wall remodeling. Experimental activation of ERC by EDP induces insulin resistance and atherosclerosis in mice. Excess EDP/ERC docking causes chemotaxis of human leukocytes and proliferation of human smooth muscle cells (SMC) and is associated with loss of arterial elasticity, atherosclerosis, increased arterial stiffness, abdominal aortic aneurysms and myocardial infarction in humans.

## Claims

1. An isolated or synthetic peptide consisting of 5-30 amino acids for use in treatment of human inflammatory disease having been provided with at least one peptide motif capable of modulating binding of human C-peptide to a human elastin receptor, wherein the peptide has at least one human elastin receptor binding motif GxxPG and has at least one amino acid Q, wherein G represents the one-letter code for the amino acid glycine, P for the amino acid proline, Q for the amino acid glutamine and x for any amino acid.

2. The peptide for use according to claim 1, consisting of 5-20 amino acids.

3. The peptide for use according to claim 1, consisting of 5-15 amino acids.

4. The peptide for use according to claim 1, consisting of 5-12 amino acids.

5. The peptide for use according to claim 1, consisting of 5-9 amino acids.

6. The peptide for use according to claim 1, consisting of any one of SEQ ID NOs: 10, 11, 12, 13, 17, 18, 19, 20, 14, 15, 16, 21, 25, 175, 3, 22, 23, 26, 176, 24, 27, 28, 29, 43, 93, and 94.

7. The peptide for use according to claim 6, consisting of any one of SEQ ID NOs: 14, 15, 16, 21, 25, 175, 3, 22, 23, 26, 176, 24, 27, 28, 29, 43, 93, and 94.

8. The peptide for use according to claim 1, being a retro-inverso variant peptide composed of D-amino acids assembled in a reverse order from that of the parent L-sequence peptides listed under SEQ ID NOs: 10, 11, 12, 13, 17, 18, 19, 20, 14, 15, 16, 21, 25, 175, 3, 22, 23, 26, 176, 24, 27, 28, 29, 43, 93, and 94.

9. The peptide for use according to claim 1, wherein the GxxPG motif is repeated at least twice, preferably thrice, optionally wherein said repeats are separated by a linker comprising one or more amino acids.

## Patentansprüche

1. Isoliertes oder synthetisches Peptid, bestehend aus 5-30 Aminosäuren zur Verwendung bei der Behandlung von humaner entzündlicher Erkrankung, das mit wenigstens einem Peptidmotiv bereitgestellt ist, fähig zum Modulieren des Bindens von humanem C-Peptid an einen humanen Elastinrezeptor, wobei das Peptid wenigstens ein humanes Elastinrezeptor-Bindungsmotiv GxxPG hat und wenigstens eine Aminosäure Q hat, wobei G den Ein-Buchstaben-Code für die Aminosäure Glycin, P für die Aminosäure Prolin, Q für die Aminosäure Glutamin und x für irgendeine Aminosäure darstellt.

2. Peptid zur Verwendung nach Anspruch 1, bestehend aus 5-20 Aminosäuren.

3. Peptid zur Verwendung nach Anspruch 1, bestehend aus 5-15 Aminosäuren.

4. Peptid zur Verwendung nach Anspruch 1, bestehend aus 5-12 Aminosäuren.

5. Peptid zur Verwendung nach Anspruch 1, bestehend aus 5-9 Aminosäuren.

6. Peptid zur Verwendung nach Anspruch 1, bestehend aus einer der SEQ-ID-Nrn: 10, 11, 12, 13, 17, 18, 19, 20, 14, 15, 16, 21, 25, 175, 3, 22, 23, 26, 176, 24, 27, 28, 29, 43, 93, und 94.

7. Peptid zur Verwendung nach Anspruch 6, bestehend aus einer der SEQ-ID-Nrn: 14, 15, 16, 21, 25, 175, 3, 22, 23, 26, 176, 24, 27, 28, 29, 43, 93, und 94.

8. Peptid zur Verwendung nach Anspruch 1, das ein Retro-Inverso-Variantenpeptid ist, zusammengesetzt aus D-Aminosäuren, aufgebaut in einer umgekehrten Reihenfolge zu der der Eltern-L-Sequenz-Peptide, gelistet unter SEQ-ID-Nrn.: 10, 11, 12, 13, 17, 18, 19, 20, 14, 15, 16, 21, 25, 175, 3, 22, 23, 26, 176, 24, 27, 28, 29, 43, 93, und 94.

9. Peptid zur Verwendung nach Anspruch 1, wobei das GxxPG-Motiv wenigstens zweimal, vorzugsweise dreimal, wiederholt ist, optional wobei die Wiederholungen durch einen Linker, umfassend eine oder mehrere Aminosäuren, getrennt sind.

## Revendications

1. Peptide isolé ou synthétique consistant en 5 à 30 acides aminés destiné à être utilisé dans le traitement d'une maladie inflammatoire humaine ayant été pourvu d'au moins un motif peptidique capable de moduler la liaison du peptide C humain à un récepteur de l'élastine humaine, où le peptide a au moins un motif de liaison au récepteur de l'élastine humaine GxxPG et a au moins un acide aminé Q, où G représente le code à une lettre pour l'acide aminé glycine, P pour l'acide aminé proline, Q pour l'acide aminé glutamine et x pour n'importe quel acide aminé.

2. Peptide destiné à être utilisé selon la revendication 1, consistant en 5 à 20 acides aminés.

3. Peptide destiné à être utilisé selon la revendication 1, consistant en 5 à 15 acides aminés.

4. Peptide destiné à être utilisé selon la revendication 1, consistant en 5 à 12 acides aminés.

5. Peptide destiné à être utilisé selon la revendication 1, consistant en 5 à 9 acides aminés.

6. Peptide destiné à être utilisé selon la revendication 1, consistant en l'une quelconque des SEQ ID NO: 10, 11, 12, 13, 17, 18, 19, 20, 14, 15, 16, 21, 25, 175, 3, 22, 23, 26, 176, 24, 27, 28, 29, 43, 93 et 94.

7. Peptide destiné à être utilisé selon la revendication 6, consistant en l'une quelconque des SEQ ID NO: 14, 15, 16, 21, 25, 175, 3, 22, 23, 26, 176, 24, 27, 28, 29, 43, 93 et 94.

8. Peptide destiné à être utilisé selon la revendication 1, étant un peptide variant rétro-inverso composé d'acides aminés D assemblés dans un ordre inverse de celui des peptides de séquence L mères répertoriés sous SEQ ID NO: 10, 11, 12, 13, 17, 18, 19, 20, 14, 15, 16, 21, 25, 175, 3, 22, 23, 26, 176, 24, 27, 28, 29, 43, 93 et 94.

9. Peptide destiné à être utilisé selon la revendication 1, dans lequel le motif GxxPG est répété au moins deux fois, de préférence trois fois, éventuellement dans lequel lesdites répétitions sont séparées par un lieur comprenant un ou plusieurs acides aminés.
